(19)

Europäisches
Patentamt

European
Patent Office

Office européen
des brevets

(11) **EP 4 556 989 A1**

(12) **EUROPEAN PATENT APPLICATION**

(43) Date of publication:
**21.05.2025 Bulletin 2025/21**

(21) Application number: **23210413.3**

(22) Date of filing: **16.11.2023**

(51) International Patent Classification (IPC):
***G02C 7/02*** *(2006.01)*

(52) Cooperative Patent Classification (CPC):
**A61B 3/103; A61B 3/0025; A61B 3/0033;
A61B 3/028; A61B 3/032; A61B 3/036; G02C 7/027**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Carl Zeiss Vision International GmbH
73430 Aalen (DE)**

(72) Inventors:
- **Leube, Alexander
  73430 Aalen (DE)**
- **Huebner, Julia
  73430 Aalen (DE)**
- **Breher, Katharina
  73430 Aalen (DE)**

(74) Representative: **Altmann Stößel Dick
Patentanwälte PartG mbB
Theodor-Heuss-Anlage 2
68165 Mannheim (DE)**

(54) **METHODS AND DEVICES FOR DETERMINING AT LEAST ONE REFRACTIVE VALUE**

(57)     The present invention relates to a method (210), a computer program and a determining device (110) for determining at least one refractive value (212) of an eye (112) of a person, a data carrier signal, a field device for generating input data (214) for determining at least one refractive value (212) of an eye (112) of a person, a remote device for generating outcome data (222) for determining at least one refractive value (212) of an eye (112) of a person, a method for generating a geometrical model (226) of at least one spectacle lens (228) for producing the at least one spectacle lens (228), and a method for producing at least one spectacle lens (228). Herein, the method (210) comprises the following steps:
a) generating input data (214) configured to comprise
- an uncorrected distance visual acuity $V_{sc}$ (216) of the eye (112) of the person;
- a meridian direction (220) of an astigmatism of the eye (112) of the person;
- a distance (140) between the eye (112) of the person and at least one visual stimulus (118) displayed to the eye (112) of the person, wherein the at least one visual stimulus (118) is simultaneously aligned in the meridian direction (220) of the astigmatism and a direction perpendicular thereto;

b) generating outcome data (222) configured to comprise
- at least one refractive value (212) of the eye (112) of the person, wherein the at least one refractive value (212) of the eye (112) of the person is determined by evaluating the input data (214),

wherein the distance (140) between the eye (112) of the person and the at least one visual stimulus (118) is determined from response of the person indicating that the at least one visual stimulus (118) has been perceived by the person to have an equivalent blur in the meridian direction (220) of the astigmatism and in the direction perpendicular thereto.

The present invention provides a fast, easy, versatile, reliable and accurate approach for determining at least one refractive value (212) of an eye (112) of a person.

Fig. 1

**Description**

The present invention relates to a method, a computer program and a determining device for determining at least one refractive value of an eye of a person, a data carrier signal, a field device for generating input data for determining at least one refractive value of an eye of a person, a remote device for generating outcome data for determining at least one refractive value of an eye of a person, a method for generating a geometrical model of at least one spectacle lens for producing the at least one spectacle lens, and a method for producing at least one spectacle lens.

Related art

Various methods and devices for determining at least one refractive value of an eye of a person are known. Herein, the terms "refraction" or "refractive" refer to a bending of incident light entering the interior of the eye via the pupil. With respect to the present invention, determining at least one refractive value of the eye of the person, particularly, comprises determining at least one value for at least one astigmatism of the eye of the person. As generally used, the term "astigmatism" corresponds to a condition in which an optical system comprising an eye of the person forms at least two individual line images of a point object, typically, as a result of a toricity of at least one refracting surface of the eye of the person. As a result thereof, the astigmatism causes that light which impinges on the eye is distributed on the retina in an unsymmetrical fashion, which may, in particular, result in at last one of a distorted or a blurred vision of the person. In general, values related to the astigmatism of the eye of the person refer to both a spatial orientation of the astigmatism, also denoted by as "axis", and an extent of the astigmatism, also denoted as "power". To completely correct the astigmatism of the eye of the person, quantifying an extent of both the axis and the power of the astigmatism of the eye of the person is particularly preferred.

Thomas W. Raasch, Spherocylindrical Refractive Errors and Visual Acuity, Optometry and Vision Science 72 (4), pp. 272-275, describes that understanding the relation between refractive error and visual acuity is complicated if astigmatic blur is present. No models are in widespread use that allow the combination of spherical and astigmatic errors for the purpose of predicting visual performance. Models for combining spherical and astigmatic errors are discussed, and predictions of these models using data from the literature are presented. Three models for combining astigmatic with spherical errors are shown to predict visual acuity performance in uncorrected myopic refractive errors. A dioptric vector addition model is shown to have advantages over other candidate models. It is possible to combine spherocylindrical refractive errors into a single value when the objective is to correlate these values with visual acuity performance.

Ralf Blendowske, Unaided Visual Acuity and Blur: A Simple Model, proposes a simple model that describes the quantitative relationship between unaided visual acuity and blur attributed to refractive errors. The standard model for describing the relationship between visual acuity and blur, as published by Raasch, see above, is used as a starting point to develop a simpler model based on heuristic arguments. The basis of Raasch's data is augmented by published findings in the range of low-level refractive errors. Sphero-cylindrical refractive errors are transformed into a single blur quantity b, also termed dioptric distance, which serves as an input in both models. The possible influence of the cylinder axis and the pupil size is not included. The quite simple model for the unaided minimum angle of resolution nicely matches available data and improves the SE of the regression by a factor of 2 in comparison to Raasch's model.

US 2019/0307324 A1 discloses a system and a method for measurement of refractive error of an eye based on subjective distance metering. The method includes: (a) displaying at least one dynamic target image of at least one sign over a display area; (b) receiving subjective feedback from the subject indicating that the subject is positioned at a maximum distance of best acuity (MDBA) from the target image, wherein the MDBA is the maximum distance in which the subject recognizes the sign; (c) measuring one or more parameter associated with distance, during the time the subject has reached the MDBA distance, using at least one sensor; (d) estimating the MDBA by estimating the distance between the eye of the subject and the display area in which the target image is displayed by using the sensor data and (e) calculating the refractive error of the eye according to the estimated MDBA and characteristics of the target image.

US 2020/0397279 A1 discloses methods, systems, and devices for measuring eye refraction without a lens, particularly for measuring eye refraction with a mobile device application. An exemplary method includes measuring a distance between a patient and a mobile device, presenting to the patient one or more visual targets sized and shaped to represent perfect vision such as by using Vernier targets or grating targets, instructing the patient to indicate whether the patient can accurately read the visual targets and, if not, to move closer to the mobile device until the patient can accurately read the visual targets, calculating a vision prescription for the patient based on the visual targets and a final distance between the patient and the mobile device, and displaying the vision prescription for the patient.

EP 3 730 038 A1 discloses a system and method for interactively measuring ocular refractive errors, addition and power of reading glasses without the need of an optical component that would change optical vergence of a target. The system can measure the distance from a user's eyes to either or both border of interval of clear vision, for one orientation or two perpendicular orientations. The measurements together with the user's age can be used to estimate sphere-cylindrical refraction, addition and power of reading glasses. The system can use different sizes, directions and colors of targets which can change with said distance or user interaction.

Problem to be solved

It is therefore an objective of the present invention, particularly in view of EP 3 730 038 A1, to provide a method, a computer program and a determining device for determining at least one refractive value of an eye of a person, a data carrier signal, a field device for generating input data for determining at least one refractive value of an eye of a person, a remote device for generating outcome data for determining at least one refractive value of an eye of a person, a method for generating a geometrical model of at least one spectacle lens for producing the at least one spectacle lens, and a method for producing at least one spectacle lens, which at least partially overcome the problems of the prior art.

It is a particular objective of the present invention to provide a fast, easy, versatile, reliable, and accurate approach for determining at least one refractive value of an eye of a person. It may be particularly an objective of the present invention to determine both meridians of an astigmatism of the eye of the person in a single measurement. Thereby, it may be desirable to determine the at least one refractive value without requiring an optometrist, an ophthalmologist, a set of measuring glasses and/or a sophisticated device, such as an autorefractive device, designated for this purpose. In particular, it may be desirable to determine the at least one refractive value in a fashion which is applicable on a global scale to all kinds of persons, whereby difficulties with communication or compliance can be reduced or avoided as far as possible.

Summary of the invention

This problem is solved by a method, a computer program and a determining device for determining at least one refractive value of an eye of a person, a data carrier signal, a field device for generating input data for determining at least one refractive value of an eye of a person, a remote device for generating outcome data for determining at least one refractive value of an eye of a person, a method for generating a geometrical model of at least one spectacle lens for producing the at least one spectacle lens, and a method for producing at least one spectacle lens comprising the features of the independent claims. Preferred embodiments, which might be implemented in an isolated fashion or in any arbitrary combination, are listed in the dependent claims or throughout the following description.

In a first aspect, the present invention relates to a method for determining at least one refractive value of an eye of a person. The method for determining at least one refractive value of an eye of a person comprises the following steps, which may be performed in the given order. A different order, however, may also be feasible. Further, two or more of the method steps may be performed simultaneously. Thereby the method steps may at least partly overlap in time. Further, the method steps may be performed once or repeatedly. Thus, one or more or even all of the method steps may be performed once or repeatedly. The method may comprise additional method steps, which are not listed herein.

The method for determining at least one refractive value of an eye of a person comprises the following steps:

   a) generating input data configured to comprise

   - an uncorrected distance visual acuity $V_{sc}$ of the eye of the person;
   - a meridian direction of an astigmatism of the eye of the person;
   - a distance between the eye of the person and at least one visual stimulus displayed to the eye of the person, wherein the at least one visual stimulus is simultaneously aligned in the meridian direction of the astigmatism and a direction perpendicular thereto;

   b) generating outcome data configured to comprise

   - at least one refractive value of the eye of the person, wherein the at least one refractive value of the eye of the person is determined by evaluating the input data,

wherein the distance between the eye of the person and the at least one visual stimulus is determined from a response of the person indicating that the at least one visual stimulus has been perceived by the person to have an equivalent blur in the meridian direction of the astigmatism and in the direction perpendicular thereto.

In general, the method for determining at least one refractive value of an eye of a person can be performed in a manual fashion in which an assistant supporting the person, preferably selected from at least one of a parent, a nurse, an optician, or an ophthalmologist, may display the at least one visual stimulus to the person as described below in more detail, wherein the at least one visual stimulus may be provided in printed form, in particular on a board, such as a cardboard or an eye chart, wherein the at least one visual stimulus is generated by using a device configured for this purpose, such as a printer or a projector. Further, the at least one refractive value of the eye of the person may be determined by evaluating the input data in a manual fashion without using a computer.

In a particularly preferred embodiment, the method for determining at least one refractive value of an eye of a person may be a computer-implemented method. As generally used, the term "computer-implemented method" refers to a method, which involves at least one apparatus, specifically a computer, particularly connected to a computer network. A plurality of apparatus may be connected, particularly for transmitting data, via a network by using at least one connection interface at any one of the apparatuses of the plurality of apparatus. The computer-implemented method may be implemented as at least one computer program that may be provided on a storage medium carrying the computer program, whereby at least one of the steps of the method, specifically at least one of steps a) or b), are performed by using the at least one computer program. Preferably any one of the steps a) and b) are performed using the at least one computer program. Alternatively, the at least one computer program may be accessible by an apparatus which may be adapted for performing the method via a network, such as via an in-house network, via internet, or via a cloud.

The present method is configured for determining at least one refractive value of an eye of a person. As generally used, the term "determining", or any grammatical variation thereof refers to a process configured for generating at least one representative result. The representative result may be determined in a process, wherein at least one step of the process may be selected from at least one of: a measurement step; an evaluation step; or a displaying step. For determining the representative result, at least one measurement may be performed. Data generated in this measurement may be evaluated. The representative result may be data retrieved in the evaluation process. The representative result may be displayed. With particular regard to the present invention, the representative result comprises outcome data comprising at least one refractive value of the eye of the person. The outcome data may be displayed on at least one monitor.

As further generally used, the terms "refraction" or "refractive" refer to a bending of incident light entering the interior of the eye of the person via a pupil, wherein the term "refractive value" refers to an observation that the incident light may, in particular owing to a form of the eye, not be focused appropriately on the retina of the eye, resulting particularly in a blurred vision of the person. The method may be performed for exactly one eye of the person simultaneously. The method may be repeated for a further eye of the person. The method may further be implemented for being performed for one eye of the person; it may, however, be possible to implement the method for both eyes of the person simultaneously. In general, the at least one refractive value of the eye of the person comprises a value of a

- a spherical power;
- a cylindrical power; and
- a cylinder axis.

Based on standard ISO 13666:2019 (referred to as "Standard" in the following), Section 3.12.2, the term "spherical power", usually abbreviated to "sphere" or "sph", refers to a value of a back vertex power of a spherical-power lens, or for a back vertex power in one of two meridians of an astigmatic-power lens, depending on a principal meridian chosen for reference. The spherical power of the eye of the person may be a value related to a "spherical equivalent". As based on the Standard, Section 3.13.2, the term "principal meridian" is one of two mutually perpendicular meridians of the astigmatic-power lens that are parallel to the two line foci. Herein, the first principal meridian algebraically has the lower vertex power, while the second principal meridian algebraically has the higher vertex power, according to the Standard, Section 3.13.3-4. As further based on the Standard, Sections 3.13.6-7, the term "cylindrical power", usually abbreviated to "cylinder" or "cyl", refers to an algebraic difference between the power of the principal meridian chosen for reference subtracted from the power of the other perpendicular meridian. As based on the Standard, Section 3.13.8, the term "cylinder axis", usually abbreviated to "cyl axis" or "axis", refers to a direction of the principal meridian of a lens whose vertex power is chosen for reference and, therefore, corresponds to the meridian direction of the astigmatism of the eye of the person.

In addition, the at least one refractive value of the eye of the person may, further, comprise at least one value related to

- an addition power.

As based on the Standard, Section 3.16.3, the term "addition power", "addition" also abbreviated to "add", refers to a difference between the vertex power of a near portion and the vertex power of a distance portion in a multifocal or power-variation lens. The addition power may further refer to a difference between the vertex power of an intermediate portion and the vertex power of a distance portion in a multifocal or power-variation lens. The addition power may further refer to a single vision lens having a vertex power that is defined for correcting vision for a distance for near vision or intermediate vision.

According to step a) of the present method for determining at least one refractive value of an eye of a person, input data is generated. As used herein, the term "generating" or any grammatical deviation thereof refers to a process of producing a set or a sequence of data, particularly by taking an initial set of data, specifically measurement data, and applying a mathematical or logical process to it. The term does not necessarily imply that a measurement process occurs. For generating input data it may be sufficient to provide the data that is comprised by the input data. Thereby, the process may make use of an algorithm or a machine learning model. As generally used, the term "algorithm" refers to a process following a predefined scheme. As generally used, the term "machine learning model" refers to a trainable computer-implemented architecture, particularly a trainable statistical model, that applies artificial intelligence to automatically determine a representative result, particularly the characteristic of the pupil. As generally used, the term "training" refers to a process of determining adjustable parameters of the machine learning model using training data for generating a trained machine learning model. The training may comprise at least one optimization or tuning process, wherein a best parameter combination is determined. The training is carried out to improve the capability of the machine learning model to determine the representative result, particularly the characteristic of the pupil, by analyzing at least a portion of the training data.

The produced set of data may be the input data. As further used herein, the term "input data" refers to a set or sequence of data that comprises information that is at least partly be evaluated by applying a mathematical or logical process to it, wherein the mathematical or logical process may be performed by a human being or, preferably, by using a computer-implemented method. In particular, the input data may be required for starting at least one step of the method, specifically the method for determining at least one refractive value of an eye of a person. The input data may be required for generating the outcome data. As already indicated above, the input data according to step a) is configured to comprise

- the uncorrected distance visual acuity $V_{sc}$ of the eye of the person;
- optionally, the corrected distance visual acuity $V_{cc}$ of the eye of the person;
- the meridian direction of the astigmatism of the eye of the person;
- the distance between the eye of the person and the at least one visual stimulus displayed to the eye of the person, wherein the at least one visual stimulus is simultaneously aligned in the meridian direction of the astigmatism and the direction perpendicular thereto.

Accordingly, the input data according to step a) is configured to comprise an uncorrected distance visual acuity $V_{sc}$ of the eye of the person. As generally used, the term "visual acuity" refers to a resolving capacity, specifically a spatial resolving capacity, of a visual system. As used herein, the visual acuity refers to a particular visual system which includes the eye of the person, specifically an ability of the eye of the person to resolve at least one fine detail with precision. In particular, the term "distance visual acuity" or "$V_{sc}$" corresponds to the visual acuity of the eye of the person to resolve the at least one fine detail in a far distance of the eye of the person without any optical correction by using an optical lens as defined below. Further, the uncorrected distance visual acuity $V_{sc}$ of the eye of the person as comprised by the input data according to step a) refers to the distance visual acuity $V_{sc}$ of the eye of the person as actually measured. As generally used, the term "measuring" or any grammatical variation thereof refers to a process of generating data, particularly comprised by the input data. The data may be obtained from at least one measurement produced in a measurement process in physical reality. For this purpose, the method may further comprise at least one measuring step. Thereby, the method may operate at least one measurement device which may, particularly, be configured for obtaining the desired result. In a particfualrly preferred embodiment, measuring the uncorrected distance visual acuity $V_{sc}$ of the eye of the person may be in line with the further standard ISO 8596:2017, Ophthalmic optics - Visual acuity testing - Standard and clinical optotypes and their presentation.

In a particularly preferred embodiment, the uncorrected distance visual acuity $V_{sc}$ may be determined by performing a measurement when the person is not using an optical lens to correct the at least one refractive value of the eye of the person. In an alternative embodiment, the person may be using an optical lens for the eye of the person while the input data is generated, particularly wherein the optical lens is having a known prescription. As generally used, the term "optical lens" refers to a visual aid, which may be used for determining and/or correcting a defective vision of the eye of the person. As generally used, the term "prescription" refers to a document that contains at least one piece of information about the optical lens. The optical lens may be the optical lens that the person is generally currently using when the person is being test by applying the method. As used herein, the term "known" refers to the fact that the prescription is available for the method and/or is considered by the method when determining outcome data. Alternatively, or in addition to using an optical lens, the person may have undergone refractive surgery. Herein, the at least one optical lens may be selected from at least one of:

- a spectacle lens;
- a contact lens; or
- an intraocular lens.

Based on the "Standard", 3.5.2, the term "spectacle lens" refers to an optical lens, which is used for determining and/or correcting a defective vision of a wearer of the optical lens, wherein the optical lens is carried in front of the eye of the wearer, thereby avoiding a direct contact with the eye of a wearer. Herein, the spectacle lens may be an optical lens the person is commonly wearing or, alternatively or in addition, a trial lens provided to the person by an optician or an ophthalmologist. As further generally used, the term "contact lens" refers to a lens placed directly on the surface of the eye of the wearer to correct visual defects. As further generally used, the term, "intraocular lens" refers to an artificial lens implanted in the eye of a wearer for correcting the defective vision. The wearer may be a person.

In a further particular embodiment, at least one of the uncorrected distance visual acuity $V_{sc}$ or the corrected distance visual acuity $V_{cc}$ of the eye of the person may be measured under cycloplegia. In this manner, values determined by auto-refraction can be determined in a more accurate fashion, particularly since non-cycloplegic auto-refraction is, generally, known to overestimate myopia and underestimate hyperopia, especially in children whose accommodative responses are active.

In a preferred embodiment, the input data according to step a) may, optionally, be configured to further comprise a corrected distance visual acuity $V_{cc}$ of the eye of the person. As used herein, the corrected distance visual acuity $V_{cc}$ of the eye of the person differs from the uncorrected distance visual acuity $V_{sc}$ of the eye of the person by taking into account the findings of Blendowske, see above. Compared to Raasch, see above, whose formula is not appropriate for small defocus or astigmatism values, the extended formula by Blendowske takes into account experimental uncertainties, normal physiological variations, age-related effects, or clinical conditions, in particular cataract, or retinal pathologies, which influence both the uncorrected distance visual acuity $V_{sc}$ and the corrected distance visual acuity $V_{cc}$ of the eye of the person in a similar fashion.

In this preferred embodiment, the corrected distance visual acuity $V_{cc}$ of the eye of the person may, in particular, be determined by using at least one of:

- a further measurement when the person is using an optical lens for the eye of the person;
- an estimated value, wherein the estimated value.

Herein, the estimated value may is, preferably, be determined by using at least one of:

∘ an age of the person;
∘ an ethnicity of the person;
∘ a gender of the person;
∘ a known value for the corrected distance visual acuity $V_{cc}$ from a known prescription for the eye of the person;
∘ a value for the corrected distance visual acuity $V_{cc}$ of $V_{cc} = 1.0$
∘ a statistical model.

For this purpose, the method may further comprise a requesting step, wherein the person may be requested to provide information related to the person in the requesting step. As used herein, the term "information related to the person" refers to information about at least one body feature of the person, more particularly, referring to at least one of the age, ethnicity and gender of the person, or to a known value for the distance visual acuity $V_{cc}$ from a known prescription for the eye of the person.

The input data according to step a) is, further, configured to comprise the meridian direction of the astigmatism of the eye of the person. For a definition of the term "astigmatism", reference can be made to the description above. Further, the term "meridian direction" refers to an orientation which is aligned to one of the meridians. Herein, the meridian direction of the astigmatism of the eye of the person may, prefereably, be determined by using at least one of:

- a known value from a known prescription;
- a further estimated value; or
- a still further measurement when the person is not using the optical lens for the eye of the person and a further response of the person indicating that the eye of the person exhibits an astigmatism.

Herein, the still further measurement may, preferably, comprise using at least one visual stimulus selected from:

- at least one line or a plurality of lines, particularly wherein the plurality of the lines is grouped in a manner according to which at least two lines of the plurality of lines are oriented in different directions;
- a sun wheel or an astigmatism dial;
- a Raubitschek figure;
- a Gabor patch;
- a grid;
- a cross;
- a Snellen E figure; or

- a Landolt C figure.

As generally used, the term "Raubitschek figure" refers to a graphical presentation comprising two paraboloid lines positioned on a rotating disc to produce an approximate arrowhead shape, wherein near the apex of the arrow the two lines approach and become parallel to each other, but without touching, wherein as the lines curve away from this point, they become increasingly separate to eventually point in opposite directions. As further generally used, the term "Gabor patch" refers to a grating, usually having a Gaussian envelope, which is known to be particularly useful as visual stimulus for the person's eye. As further generally used, the terms "Snellen E figure" and "Landolt C figure" each refer to a standardized symbol used for testing vision.

For a purpose of performing the still further measurement, a known device which is configured for determing the meridian direction of the astigmatism of the eye of the person may, preferably, be used, espcially selected from at least one of:

- an auto-refraction apparatus;
- a wavefront aberrometer.

However, using a different device configured for determing the meridian direction of the astigmatism of the eye of the person may also be feasible.

The input data according to step a) is, further, configured to comprise the distance between the eye of the person and the at least one visual stimulus displayed to the eye of the person. As generally used, the term "displaying" or any grammatical deviation thereof refers to a presentation of at least one of an image, an item, a text, or a video, particularly the at least one visual stimulus, by using at least one board or screen. As already indicated above, the term "board" refers to a two-dimensional element, such as a cardboard or an eye chart, comprising on a surface on which at least one visual stimulus is generated by using a device configured for this purpose, such as a printer or a projector.

As further generally used, the term "screen" refers to an electronic visual display device designated for the presentation of at least one of an image, an item, text, or a video transmitted electronically. In particular, the at least one screen may be comprised by at least one of:

- a smartphone;
- a smart watch;
- a smart glass;
- an augmented reality glass;
- a virtual reality glass;
- a desktop computer;
- a laptop;
- a tablet; or
- a smart television set.

As generally used, the term "smartphone" refers to a mobile phone having computer functionalities and connectivity and may, additionally, comprise at least one sensor and/or at least one actuator, for example at least one vibration motor. As generally used, the term "smart watch" refers to an electronic wristwatch that has computer functionalities and connectivity and may, additionally, comprise at least one sensor and/or at least one actuator, for example at least one vibration motor. As generally used, the term "smart glasses" refers to wearable spectacles having computer functionalities and connectivity, and may, additionally, add information onto at least one optical lens alongside to what the wearer sees. As further generally used, the term "augmented reality glass" refers to at least one controllable optical lens, preferably a pair of controllable optical lenses, being configured to give a wearer access to a augmented reality. As further generally used, the term "virtual reality headset" refers to a head-mounted display designed to give a wearer access to a virtual reality. As generally used, the term "desktop computer" refers to a computer in a housing shape suitable for use as a workstation computer on desks. As generally used, the term "laptop" refers to a special type of computer having a screen being movably attached to a housing, wherein the screen can be folded onto the housing. As generally used, the term "tablet" refers to a portable, flat touch-screen computer. As generally used, the term "smart television" refers to a television set, additionally, comprising computer functionalities and connectivity.

As further used herein, the term "visual stimulus" refers to a graphical presentation of an item, particularly an item that is known or reasonably to be expected by the person skilled in the art to be considerably suitable for determining at least one refractive value of an eye of a person. The visual stimulus may particularly be suitable if it is perceptible by the person, especially due to a contrast between the visual stimulus and a background that allows the eye of the person to distinguish between the visual stimulus and the background. As already indicated above, the at least one visual stimulus is displayed in a manner that it is simultaneously aligned in the meridian direction of the astigmatism of the eye of the person and a direction perpendicular thereto. For this purpose, the at least one visual stimulus may, preferably, comprise at least one of:

- a plurality of lines, particularly wherein the plurality of the lines is grouped in a manner according to which at least two lines of the plurality of lines are oriented in the meridian direction of the astigmatism and the direction perpendicular thereto;
- a sun wheel or an astigmatism dial;
- a Raubitschek figure;
- at least two Gabor patches;
- a grid;
- a cross; or
- a Snellen E figure.

For the terms "Raubitschek figure", "Gabor patch" and "Snellen E figure", reference can be made to the definitions as provided above.

As generally used, the term "distance" refers to the shortest connection between two points. As used herein, the distance refers to the shortest connection between the eye of the person and a location of the at least one visual stimulus. With particular regard to the invention the shortest connection may consider the optical path the light impinging on the eye is propagating from the at least one visual stimulus to the eye. For determining the value of the distance between the eye of the person and at least one visual stimulus displayed to the eye of the person to be used for determining the at least one refractive value of the eye of the person, the distance may be altered until the person indicates that the at least one visual stimulus has been perceived by the person to have an equivalent blur in the meridian direction of the astigmatism and in the direction perpendicular thereto. In particular, the distance between the eye of the person and the at least one visual stimulus displayed to the eye of the person may be altered by at least one of:

- moving the eye of the person towards the at least one board or screen;
- moving the at least one board or screen towards the eye of the person; or
- altering the geometry of the field of view of the eye of the person between the eye and the at least one visual stimulus, particularly by using at least one of:

    ○ at least one optical lens; or
    ○ at least one mirror;

- altering at least one of an augmented reality or a virtual reality.

In a particular embodiment, altering the distance between the eye of the person and the at least one visual stimulus may be motivated by at least one command provided to the person, wherein the at least one command comprises a request to the person to alter the distance with regard to the at least one visual stimulus. As generally used herein, the term "command" refers to a request provided to the person to perfrom a particular process indicated by using at least one of a signal or a piece of textual information. Preferably, the at least one command may be provided to the person by at least one of:

- a visual signal, particularly at least one of piece of textual information or a gesture;
- an audio signal, particularly a voice output or a vibration, specifically generated by using a loudspeaker;
- a tactile signal.

As used herein, the term "visual signal" refers to a sign being perceptible by a sense of sight. The term "audio signal" is refers to a sound perceptible by an sense of hearing. In particular, the piece of textual information may be selected from:

- a command requesting the person to move nearer to or further away from the at least one visual stimulus, in particular the at least one board or screen displaying the at least one visual stimulus; or
- a command requesting the person to maintain a position with respect to the at least one visual stimulus, in particular the at least one board or screen displaying the at least one visual stimulus.

However, using a different piece of textual information may also be conceivable.

The distance between the eye of the person and the at least one visual stimulus displayed to the eye of the person may, particularly in a monotonous fashion, be altered by decreasing or increasing the distance. As generally used, the term "monotonous" refers to a uniform and/or consistent motion. The motion may considered as monotonous when a direction of the motion is constant and/or remains, particularly unaltered. Thereby the distance between the eye of the person and the at least one visual stimulus may be altered by being constantly decreased or increased. The direction in which the distance alters may be considered as remaining constant. The speed of the alteration of the distance may fluctuate. Herein, the distance between the eye of the person and the at least one visual stimulus displayed to the eye of the person may decrease or increase from a starting position. In a particular embodiment, the person may hold the at least one board or screen in at least one hand and may alter the distance, particularly by decreasing or increasing the distance, between the eye of the person and the at least one visual stimulus displayed to the eye of the person. Alternatively, or in addition, the at least one board or screen may be fixedly positioned, particularly on a stable ground, such as a floor or a table, or wall in front of the person while the person may be altering the distance between the eye of the person and the at least one visual stimulus displayed to the eye of the person.

As indicated above, the distance between the eye of the person and the at least one visual stimulus is determined at a point in time, especially at a single point in time, of a response which is received from the person, wherein the response of the person at the point in time, especially at the single point in time, indicates that the at least one visual stimulus has been perceived by the person as having an equivalent blur in the meridian direction of the astigmatism and in the direction perpendicular thereto. As used herein, the term "at a point in time" refers to a specific moment, especially a single moment, at which the person recognizes that a portion of the at least one visual stimulus which is displayed in the meridian direction of the astigmatism exhibits a same amount of blur as a further portion of the at least one visual stimulus which is displayed in the direction perpendicular thereto.

In general, a visual perception of the at least one visual stimulus by the person may be clear or having a blur. As generally used, the terms "clear" or "sharp" refer to a state in which an object is spatially resolved in an optimal fashion with regard to a spatially resolving capacity of a visual system. Accordingly, the person considers the at least one visual stimulus as clear, when the person considers that the ability of the eye of the person to resolve at least one fine detail in the at least one visual stimulus is met as good as possible. In contrast hereto, the terms "blurred" or "having a blur" refer to a contrasting state in which the object is not spatially resolved in an optimal fashion with regard to the spatially resolving capacity of a visual system.

Accordingly, the person may consider the at least one visual stimulus as having a blur, when the person considers that the ability of the eye of the person to resolve at least one fine detail in the at least one visual stimulus is not met. In particular, the at least one visual stimulus may be considered by the person as having a blur, when the person cannot identify a highest number of details in the at least one visual stimulus under consideration of the ability of the person. Herein, an absolute amount of details that can be identified in the at least one stimulus, in general, varies between different persons, especially owing to the visual acuity of the eye of each person.

Surprisingly, it has been found that determining the distance between the eye of the person and the at least one visual stimulus at a point in time, especially a single point in time, of a response received from the person, wherein the response of the person at the point in time, especially at the single point in time, indicates that the at least one visual stimulus has been perceived by the person as having an equivalent blur in the meridian direction of the astigmatism and in the direction perpendicular thereto, provides considerably reliable and accurate results that can be used for determining at least one refractive value of an eye of a person in a fast, easy, versatile manner. Without wishing to be bound by theory, the eye of a person has, in general, a considerable ability to compare fine details or contrast levels in two images or in two portions of a single image being simultaneously displayed to the eye of the person in a more reliable and accurate manner compared to two images or two portions of a single image being consecutively displayed to the eye of the person. In other words: It has been found that it is much easier for the eye of the person to determine a point in time at which two images or two portions of a single image exhibit the same fine details or have the same contrast level.

This advantage particularly arises in view of the disclosure of EP 3 730 038 A1, which uses a different method, according to which the at least one refractive value of an eye of a person is determined by using a first distance and a second distance between the eye of the person and the at least one visual stimulus at a first point in time and a second point in time of a response by the person, respectively. Herein, the first point in time indicates that the person perceives the at least one visual stimulus in clear vision in a first meridian direction, while the second point in time indicates that the person perceives the at least one visual stimulus in clear vision in a second meridian direction being perpendicular to the first meridian direction. However, absolute values of the fine details or the contrast level depend on an individual visual perception of the person with regard to sharpness and blur, which may, moreover, change over time at consecutive points in time, such as the first point in time and the second point in time.

In a particularly preferred embodiment, wherein the point in time of the response received from the person may, preferably, be determined by using at least one of:

- an indication of the person or a further person supporting the person, preferably selected from at least one of a parent, a nurse, an optician, or an ophthalmologist, wherein the indication is, particularly, provided by generating at least one of:

○ a visual signal, particularly a gesture;
○ an audio signal, particularly a voice input, specifically provided by using a microphone;
○ a tactile signal, particularly by using at least one of:

- a keypad; or
- a touch pad;

- a continuous distance measurement;
- a staircase method;
- a forced choice method.

As used herein, the term "indication" refers to a detail or an information directly and/or indirectly related to the feature the indication is about, wherein the indication may be the feature itself or a reference to the feature. With regard to the terms "visual signal" and "audio signal", reference can be made to the definitons as provided above. The term "tactile signal" refers to an impression that is perceived by a haptic sensation, for example haptic sensations such as, but not limited to, tickling, a touch, a movement, a vibration, a temperature, a pressure and/or a tension. As generally used, the term "staircase method" refers to method involving upward and downward movements of a size or spatial characteristic of the visual stimulus and/or the distance. Thereby, a test-retest variability may be improved. As further generally used, the term "forced choice method" refers to a process which employs a forced choice principle. Herein, the term "forced choice principle" refers to a trial procedure in which the person being tested is necessarily required to give an indication related to or about a subject to be tested. The person may necessarily give an indication even in case that the person is unsure. Thereby, the person may have to give a guessed indication rather than an indication that the person is unsure. Typical indications may be answers selected from "yes" and "no", "left" and "right", "or "nearer" and "further away". However, using a different method for determining the point in time of the response received from the person may also be conceivable.

As generally used, the term "a continuous distance measurement" refers to a test procedure that uses a continuous distance measurement for positioning the person, particularly as close as possible to a visual stimulus in a manner that the visual stimulus may be seen by the person, in particular as spatially resolved in an optimal fashion. In particular, the at least one distance between the eye of the person and the at least one visual stimulus may be continuously measured. As generally used, the term "continuous" or any grammatical variation thereof refers to an ongoing, uninterrupted, or uniformly performed process. For measuring the distance continuously, the distance measurement may be repeated after a predetermined time interval between each repetition. Alternatively, the distance may be measured all the time, espcially as often as technically possible. The size of the at least one visual stimulus displayed to the eye of the person on a screeen may be adjusted depending on the at least one distance continuously measured between the eye of the person and the at least one visual stimulus. The size of the at least one visual stimulus displayed to the eye of the person on a screen may be adjusted in a manner that the person perceives the at least one visual stimulus in the same size independently from the at least one distance continuously measured between the eye of the person and the at least one visual stimulus.

As indicated above, the distance between the eye of the person and the at least one visual stimulus is determined at a point in time, especially at a single point in time, of a particular response received from the person as described elsewhere herein. For this purpose, a distance measuring device may, preferably, be used. As generally used, the term "distance measuring device" refers to an electronic component configured for generating data, in particular distance measurement data, as comprised by the input data. The distance measurement data may comprise at least one value of the at least one distance. Preferably, the distance measuring device may be selected from at least one of:

- an image-capturing device;
- a sensor-based capturing device, particularly selected from at least one of:

○ an infra-red emitter and detector;
○ a LiDAR module; or
○ a Bluetooth module;

- an ultra-wideband device, particularly comprising at least two communication devices; or
- an independent measurement device, particularly wherein the measurement is performed in a manual fashion by the person, specifically wherein the independent measurement device is a ruler.

As generally used, the term "sensor-based capturing device" refers to at least one of a distance sensor or a proximity sensor configured to detect a plurality of signals by using a sensor, particularly for measuring the at least one distance. The detected sensor signal may comprise information related to the at least one distance. The distance measuring device may, additionally or alternatively, be at least one "image-capturing device" configured for an image-based measurement of the at least one distance, particularly a camera, specifically of a smartphone, more particularly a back and/or a front camera of the smartphone. For measuring the at least one distance, the at least one distance measuring device, particularly the at least one image-capturing device, more particularly the respective camera, may be calibrated by using standard calibration methods known to the person skilled in the art. The sensor-based capturing device may be an emitter-detector device. As generally used, the term "emitter-detector device" refers to a distance sensor and/or a proximity sensor that emit at least one signal by using at least one emitter and detecting this, particularly reflected, at least one signal by using at least one detector. The difference in the emitted signal and the detected signal may comprise information related to the distance, particularly for determining the at least one distance. As further generally used, the term "LiDAR scanning module" refers a device suitable for determining the at least one distance by targeting an object or a surface with a laser and measuring the time for the reflected light returning to the receiver. As further generally used, the term "independent measurement device" refers to a measurement device that is not comprised by any further device as used in the present method. In particular, the independent measurement device may be an analog device, preferably a non-electrical device, such as a ruler, especially a pocket rule, an expanding folding rule, or a measuring rod. However using a pocket laser rangefinder, also denoted as a "laser telemeter", may also be feasible. The results of the measurements comprising the use of the independent measurement device may be input to the method by using an input device and/or a connection interface.

In a particular embodiment, the present method may, further, comprise monitoring an accommodation of the eye of the person, in particular, when the person is viewing at the at least one visual stimulus. For this purpose, photoretinoscopy may, particularly, be used; however using a different method for monitoring the accommodation of the eye of the person may also be feasible. Monitoring the accommodation of the eye of the person may ensure that a line of sight from the eye of the person may actually be directed towards at least one visual stimulus displayed to the eye of the person.

A further eye of the person for which the at least one refractive value is not determined may be shielded from the at least one visual stimulus displayed to the eye of the person. As used herein, the term "shielding" or any grammatical variation thereof refers to the further eye being prevented from perceiving the at least one visual stimulus, particularly by blocking or redirecting at least a part or the full field of view of the further eye so that light originating from the at least one visual stimulus cannot imping on the eye. The term "field of view" as used herein refers to an extent of the observable world that is observable, particularly by the further eye of the person. The field of view may be considered as a solid angle through which an eye, particularly the further eye, is sensitive to the at least one visual stimulus. The further eye of the person may be shielded by at least one of:

- closing the further eye; or
- covering the further eye, specifically by using a cover, preferably a cardboard or a hand of the person or of a further person.

However, further embodiments may also be feasible. In a further embodiment, images transmitted via different imaging beam paths assigned to the right eye and to the left eye, respectively, may be separated, preferably by using ast leat one of an optical filter, a grating, an optical lens, an optical prism, an optical diaphragm, or a mirror, in particular for a purpose of stereoscopic image transmission such as used for augmented reality glass or virtual reality.

According to step b), outcome data is generated. As used herein, the term "outcome data" refers to a set or sequence of data that comprises information determined in at least one step of a method, specifically the method for determining at least one refractive value of the eye of a person. The outcome data may comprise the representative result, particularly determined by the method for determining at least one refractive value of an eye of a person. For a generation of the outcome data, the input data may be evaluated. As already indicated above, the outcome data according to step b) is configured to comprise

- at least one refractive value of the eye of the person, wherein the at least one refractive value of the eye of the person is determined by evaluating the input data.

As generally used, the term "evaluating" or any grammatical variation thereof refers to a process of interpreting or analyzing pieces of data, specifically for obtaining at least one representative result. According to the present method, the input data may be evaluated for a purpose of determining the at least one refractive value of the eye of the person.

In a particularly preferred embodiment, determining the at least one refractive value of the eye of the person by evaluating the input data may comprise

- determining a value of a blur vector by using the uncorrected distance visual acuity $V_{sc}$ of the eye of the person and, preferably, the corrected distance visual acuity $V_{cc}$;
- determining a spherical equivalent by using the at least one distance determined from the response of the person indicating that the at least one visual stimulus has the equivalent blur in the meridian direction of the astigmatism and in the direction perpendicular thereto; and

- determining the spherical power and the cylindrical power by using the value of the blur vector and the spherical equivalent.

Herein, the value of the blur vector is defined by using equation (1):

$$(blur\ vector)^2 = (sph + 0.5 * cyl)^2 + (0.5 * cyl)^2, \qquad (1)$$

wherein $sph$ denotes the spherical power and $cyl$ the cylindrical power.

Further, determining the value of the blur vector comprises using equations (2) and (3):

$$(blur\ vector)^2 = \left(\frac{1}{V_{rel}-1}\right)^2, \qquad (2)$$

wherein

$$V_{rel} = \frac{V_{sc}}{V_{cc}}, \qquad (3)$$

wherein $V_{sc}$ denotes the uncorrected distance visual acuity of the eye of the person, and wherein $V_{cc}$ denotes the corrected distance visual acuity of the eye of the person.

For determining the spherical equivalent, equation (4), wherein

$$spherical\ equivalent = 1/d_{DEMB}, \qquad (4)$$

is used, wherein $d_{DEMB}$ denotes the distance determined from the response of the person indicating that the at least one visual stimulus has the equivalent blur in the meridian direction of the astigmatism and in the direction perpendicular thereto.

Finally, determining the spherical power $sph$ and the cylindrical power $cyl$ comprises using equations (5) and (6):

$$cyl = 2 * \sqrt{(blur\ vector)^2 + (spherical\ equivalent)^2}, \qquad (5)$$

$$sph = spherical\ equivalent - 0.5 * cyl. \qquad (6)$$

As a result thereof, the the spherical power $sph$ and the cylindrical power $cyl$ have been determined from using the value of the blur vector and the spherical equivalent, as determined from the input data as defined above.

The outcome data may be presented by using an output interface configured for presenting the outcome data to the at least one person and/or to a further person supporting the person, preferably selected from at least one of a parent, a nurse, an optician, or an ophthalmologist. Preferably the outcome data may be presented on at least one monitor, preferably as at least one of a diagram or a graphic presentation showing the at least one refractive value of the eye of the person. In a particular embodiment, the at least one screen which is used for displaying the at least one visual stimulus may, also, be used as the at least one monitor; however, using a different momitor may also be feasible. Alternatively or in addition, a loudspeaker may be used as output interface.

Step a) of the present method may define a measurement cycle. A plurality of measurement cycles may be performed. Step b) of the present method may define an evaluation cycle. A plurality of measurement cycles may be performed, wherein in each measurement cycle the at least one distance may be measured, wherein any one of the at least one measured distance may be considered in the evaluation cycle, particularly in a final evaluation cycle.

According to a further aspect of the present invention, a computer program for determining at least one refractive value of an eye of a person is disclosed, comprising instructions which, when the program is executed by a computer, cause the computer to carry out the method for determining at least one refractive value of an eye of a person. As generally used, the term "computer program" refers to at least one executable instruction for at least one programmable apparatus, specifically a computer, preferably a sequence of executable instructions, for processing and/or solving of at least one function and/or at least one task and/or at least one problem by using at least one programmable apparatus or device, specifically a computer, preferably for performing some or all steps of any one of the methods as described within the present invention. Typically, instructions are combined to a computer program code and/or provided in a programming language. A computer program is typically processed by using a processing device comprised by the at least one computer. For this purpose, the computer program may be running on the computer. The computer program code may be provided on a data storage medium or a separate device such as an optical storage medium, e.g., on a compact disc, directly on a computer or data processing device, or via a network, such as via an in-house network or via internet.

According to a further aspect, the present invention relates to a data carrier signal carrying outcome data which are generated by a method for determining at least one refractive value of an eye of a person which comprises generating the outcome data. As generally used, the term "data carrier signal" refers to an electronic signal comprising information. The data carrier signal may be provided on a computer-readable storage medium. As used herein, the term "computer-readable storage medium" specifically may refer to a non-transitory data storage means, such as a hardware storage medium having stored thereon computer-executable instructions.

For further details concerning the computer program and the data carrier signal, reference can be made to the method for determining at least one refractive value of an eye of a person as disclosed elsewhere herein.

According to a further aspect, the present invention relates to a field device for generating input data for determining at least one refractive value of an eye of a person, wherein the field device is configured to carry out at least the following step of a method for determining at least one refractive value of an eye of a person, comprising the following steps:

a) generating input data configured to comprise

- an uncorrected distance visual acuity $V_{sc}$ of the eye of the person;
- a meridian direction of an astigmatism of the eye of the person;
- a distance between the eye of the person and at least one visual stimulus displayed to the eye of the person, wherein the at least one visual stimulus is simultaneously aligned in the meridian direction of the astigmatism and a direction perpendicular thereto;

wherein the input data are transmitted to a remote device by using a connection interface for determining at least one refractive value of the eye of the person by evaluating the distance between the eye of the person and the at least one visual stimulus as determined from a response of the person indicating that the at least one visual stimulus has been perceived by the person to have an equivalent blur in the meridian direction of the astigmatism and in the direction perpendicular thereto.

As used herein, the term "field device" refers to a device that is configured for generating the input data. The person may have access to the field device and/or the person may use the field device, particularly for generating the input data. Alternatively a further person may use the field device on the person, particularly for generating the input data. The field device may be operable without a remote devices described below in more detail. As used herein, the term "remote device" refers to a device that is used for generating the outcome data. The person may not have access to the field device and/or the person may not or may only indirectly use the field device, particularly for generating the outcome data. The remote device may be owned, used and/or controlled by a third party, for example a company or a further person. The remote device may be operable without the field device. The field device may transmit the input data to a specific remote device depending on at least one circumstance, such as a date, a day, a low load of the specific remote device, particularly in comparison to at least one alternative remote device, low costs for running the specific remote device, particularly in comparison to at least one alternative remote device, and so on. The specific remote device may not be directly selected by the field device but rather a further device may specify to which specific remote device the input data may be transmitted from the field device. The generation of the outcome data may involve a use of several different entities of the remote device.

At least one entity may generate intermediate data and transmit the intermediate data by using a connection interface to at least one further entity. The outcome data may be transmitted from the remote device to the field device, particularly by using a data carrier signal carrying the outcome data. Thereby, the person may be able to analyze the outcome data, specifically the at least one refractive value of the eye of the person. As generally used, the term "connection interface" refers an item or an element configured for transmitting information or data, particularly input data or outcome data. In particular, the connection interface may be configured for transmitting information from a computational device, e.g. a computer, such as to forward input data or output data, e.g. onto a further device. Additionally or alternatively, the connection interface may be configured for transmitting information onto a computational device, e.g. onto a computer, such as to receive information. In a particular embodiment, the connection interface may be configured for both transmitting or exchanging information. The connection interface may, preferably, be selected from at least one of:

- a network interface controller; or
- a transmitter;

however using a different kind of connection interface may also be feasible. As generally used, the term "network interface controller" refers to a computer hardware component configured to connect a computer to a computer network. As generally used, the term "transmitter" refers to an electronic device, which produces electromagnetic waves with an antenna. More particular, the connection interface may provide a data transfer connection, e.g. Bluetooth, NFC, or inductive coupling. As an example, the connection interface may be or comprise at least one of a network or internet port, a USB-port, and a disk drive.

In particular, the field device may comprise at least one processing device, which is configured for generating input data configured. However, the processing device may, as described below in more detail, be configured for perfroming at least one further task. As generally used, the term "processing device" refers to an arbitrary logic circuitry configured for performing basic operations of a computer or system, and/or, generally, to a device which is configured for performing calculations or logic operations. Herein, the processing device may be a single device. As an alternative, the processing device device may comprise a plurality of elements which are located on more than a single location, wherein at least two elements located on at least two different locations are configured to communicate with each other by using at least one connection interface, especially at least one connection interface as described elsewhere herein in more detail. In particular, the processing unit may be configured for processing basic instructions that drive the computer or system. As an example, the processing unit may comprise at least one arithmetic logic unit (ALU), at least one floating-point unit (FPU), such as a math co-processor or a numeric coprocessor, a plurality of registers, specifically registers configured for supplying operands to the ALU and storing results of operations, and a memory, such as an L1 and L2 cache memory. In particular, the processing unit may be a multi-core processor. Specifically, the processing unit may be or may comprise a central processing unit (CPU). Additionally or alternatively, the processing unit may be or may comprise a microprocessor, thus specifically the processing unit's elements may be contained in one single integrated circuitry (IC) chip. Additionally or alternatively, the processing unit may be or may comprise one or more application-specific integrated circuits (ASICs) and/or one or more field-programmable gate arrays (FPGAs). The processing unit specifically may be configured, such as by software programming, for performing one or more evaluation operations.

Further, the field device may comprise at least one distance measuring device, in particular a distance measuring device as described above or below in more detail. As indicated above, the distance measuring device may, preferably, be used for determining the distance between the eye of the person and the at least one visual stimulus at a point in time, especially at a single point in time, of a particular response received from the person as described elsewhere herein.

The field device may, preferably, be selected from at least one of:

- a smartphone;
- a smart watch;
- a smart glass;
- an augmented reality glass;
- a virtual reality glass;
- a desktop computer;
- a laptop;
- a tablet; or
- a smart television;

however using a different kind of field device may also be feasible.

According to a further aspect, the present invention relates to a remote device for generating outcome data for determining at least one refractive value of an eye of a person, wherein the remote device is configured to carry out at least the following step of a method for determining at least one refractive value of an eye of a person, wherein the remote device is receiving input data provided by a field device by using a connection interface, wherein the method is comprising the following step:

b) generating outcome data configured to comprise

- at least one refractive value of the eye of the person, wherein the at least one refractive value of the eye of the person is determined by evaluating the input data,

wherein the input data comprises a distance between the eye of the person and the at least one visual stimulus which is determined from a response of the person indicating that the at least one visual stimulus has been perceived by the person to have an equivalent blur in the meridian direction of the astigmatism and in the direction perpendicular thereto.

In particular, the remote device may comprise a processing device, which is at least configured for generating the desired outcome data. For further details with regard to the processing device, reference may be made to the description above and below. Espeically for this purpose, the remote device may, preferably, be selected from at least one of:

- a server; or
- a cloud server;

however using a different kind of remote device may also be feasible. As generally used, the term "server" refers to device for performing at least one task for a further device connected to the server for transmitting data, particularly connected by a network such as the internet or a private network. As generally used, the term "cloud server" refers to a server that is accessible via a public network such as the internet. A cloud server may particularly be owned by a third party and offer third party service such as evaluating the input data for generating the outcome data and thereby determining the at least one refractive value of the eye of the person.

According to a further aspect, the present invention relates to a determining device for determining at least one refractive value of an eye of a person, wherein the device is configured to carry out a method for determining at least one refractive value of an eye of a person, wherein the method comprises the following steps:

a) generating input data configured to comprise

- an uncorrected distance visual acuity $V_{sc}$ of the eye of the person;
- a meridian direction of an astigmatism of the eye of the person;
- a distance between the eye of the person and at least one visual stimulus displayed to the eye of the person, wherein the at least one visual stimulus is simultaneously aligned in the meridian direction of the astigmatism and a direction perpendicular thereto;

b) generating outcome data configured to comprise

- at least one refractive value of the eye of the person, wherein the at least one refractive value of the eye of the person is determined by evaluating the input data,

wherein the distance between the eye of the person and the at least one visual stimulus is determined from a response of the person indicating that the at least one visual stimulus has been perceived by the person to have an equivalent blur in the meridian direction of the astigmatism and in the direction perpendicular thereto.

In a particularly preferred embodiment, the determining device may comprise both a field device and a processing device, which may be configured for both generating the input data and the outcome data. For further details with regard to the field device and the processing device, reference may be made to the description above and below.

The determining device may, preferably, be at least one of:

- a smartphone;
- a smart watch;
- an augmented reality glass;
- a virtual reality glass;
- a desktop computer;
- a laptop;
- a tablet; or
- a smart television;

however using a different kind of remote device may also be feasible.

For further details concerning the field device for generating input data for determining at least one refractive value of an eye of a person, the remote device for generating outcome data for determining at least one refractive value of an eye of a person, and the determining device for determining at least one refractive value of an eye of a person, reference can be made to the other devices as well as to the method for determining at least one refractive value of an eye of a person as disclosed elsewhere herein.

According to a further aspect, the present invention relates to a method for generating a geometrical model of at least one spectacle lens for producing the at least one spectacle lens, wherein generating the geometrical model of the at least one spectacle lens comprises

- determining the data related to the at least one refractive value by carrying out a method for determining at least one refractive value of an eye of a person in a vision testing procedure, wherein the vision testing procedure comprises at least one test cycle, preferably at least two subsequent test cycles, wherein each test cycle comprises at least the following steps:

a) generating input data configured to comprise

- an uncorrected distance visual acuity $V_{sc}$ of the eye of the person;
- a meridian direction of an astigmatism of the eye of the person;
- a distance between the eye of the person and at least one visual stimulus displayed to the eye of the person, wherein the at least one visual stimulus is simultaneously aligned in the meridian direction of the astigmatism and a direction perpendicular thereto;

b) generating outcome data configured to comprise

- at least one refractive value of the eye of the person, wherein the at least one refractive value of the eye of the person is determined by evaluating the input data,

wherein the distance between the eye of the person and theat least one visual stimulus is determined from a response of the person indicating that the at least one visual stimulus has been perceived by the person to have an equivalent blur in the meridian direction of the astigmatism and in the direction perpendicular thereto; and

- generating the geometrical model of the at least one spectacle lens for the eye of the person by using the outcome data.

As used herein, the term "vision testing procedure" refers to a procedure in which the at least one refractive value of an eye of a person is determined. The at least one vision testing procedure may comprise the steps of the method for determining at least one refractive value of an eye of a person as described elsewhere herein.

According to a further aspect, the present invention relates to a method for producing at least one spectacle lens, wherein producing the at least one spectacle lens comprises processing at least one lens blank, wherein the processing of the at least one lens blank is performed by using the geometrical model of the at least one spectacle lens as generated by a method for generating the geometrical model of the at least one spectacle lens for producing the at least one spectacle lens as disclosed elsewhere herein.

For further details concerning the method for generating a geometrical model of at least one spectacle lens and the method for producing at least one spectacle lens, reference can be made to the method for determining at least one refractive value of an eye of a person as disclosed elsewhere herein.

With respect to the prior art, the device exhibits the following advantages. The invention provides a fast, easy, versatile, reliable and accurate approach for determining at least one refractive value of an eye of a person. In particular, the present invention allows determining both meridians of an astigmatism of the eye of the person in a single measurement. Further, the method for measuring the at least one refractive value an eye of a person as disclosed herein does not require the use of refractive correction glasses during the measurement process. Further, the present invention provides a sensor-based distance measurement in real time having a reduced error, particularly with regard to the distance measurement.

In contrast hereto, US 2019/0301324 A1 discloses performing a measurement of the uncorrected visual acuity and a single cylindric meridian in different distances by using different sizes of the visual stimulus, wherefrom the sphero-cylindrical refraction is determined. Such akind of measurement is rather tedious, particularly since various measurements at different distances are required.

In further contrast hereto, US 2020/0397279 A1 discloses performing a measurement of the spherical equivalent by using visual acuity measurement at different distances. As proposed, only the spherical equivalent is determined here, but no individual sphero-cylindrical component.

As already indicated above, EP 3 730 038 A1 discloses an adjustment of a distance between a screen, in particular a smartphone, and a person in two astigmatism meridians within borders of the interval of clear vision and, subsequently, determining both astigmatism distance points and, therefrom, the sphero-cylindrical refraction. However, using two different points in time indicating that the person perceives the at least one visual stimulus in clear vision in two different meridian directions, disadvantaegeously, requires perceiving absolute values of fine details or contrast levels by the person at two different points in time. Since the determining of both astigmatism distance points depends on an individual visual perception of the person with regard to sharpness and blur, which may, moreover, change over time at consecutive points in time, this method can, inherently, not be as reliable and accurate compared to the approach as disclosed herein, which is, moreover, faster compared to the method of EP 3 730 038 A1, particularly owing to the feature of determining both meridians of an astigmatism of the eye of the person in a single measurement.

In addition, the input data which are provided according to the approach as disclosed herein is independent from varuíous characteristics of the at least one visual stimulus, preferably, selected from at least one of:

- a type of the at least one visual stimulus;
- a color of the at least one visual stimulus;
- a spatial frequency of the at least one of the visual stimulus;
- a size of the at least one of the visual stimulus;
- a contrast level of the at least one of the visual stimulus;
- a polarization of the at least one of the visual stimulus;
- a flicker frequency of the at least one of the visual stimulus;
- a display time of the at least one of the visual stimulus; or
- a shape of the at least one of the visual stimulus.

As a result thereof, the present invention can rather easily performed in a highly versatile fashion.

As used herein, the terms "have", "comprise" or "include" or any arbitrary grammatical variation thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

Summarizing, the following Embodiments are particularly preferred within the scope of the present invention:

Embodiment 1. A method for determining at least one refractive value of an eye of a person, the method comprising the following steps:

a) generating input data configured to comprise

- an uncorrected distance visual acuity $V_{sc}$ of the eye of the person;
- a meridian direction of an astigmatism of the eye of the person;
- a distance between the eye of the person and at least one visual stimulus displayed to the eye of the person, wherein the at least one visual stimulus is simultaneously aligned in the meridian direction of the astigmatism and a direction perpendicular thereto;

b) generating outcome data configured to comprise

- at least one refractive value of the eye of the person, wherein the at least one refractive value of the eye of the person is determined by evaluating the input data,

wherein the distance between the eye of the person and the at least one visual stimulus is determined from a response of the person indicating that the at least one visual stimulus has been perceived by the person to have an equivalent blur in the meridian direction of the astigmatism and in the direction perpendicular thereto.

Embodiment 2. The method according to the preceding Embodiment, wherein the method is

- performed in a manual fashion by the person; or
- a computer-implemented method.

Embodiment 3. The method according to the preceding Embodiment, wherein the the person is accompanied by a further person supporting the person, preferably selected from at least one of a parent, a nurse, an optician, or an ophthalmologist.

Embodiment 4. The method according to any one of the preceding Embodiments, wherein the at least one refractive value of the person comprises a value of a

- a spherical power;
- a cylindrical power; and
- a cylinder axis,

wherein the cylinder axis corresponds to the meridian direction of the astigmatism of the eye of the person.

Embodiment 5. The method according to the preceding Embodiment, wherein the refractive value of the eye of the person further comprises a value of an

- an addition power, particularly related to a viewing zone for near vision or intermediate vision.

Embodiment 6. The method according to any one of the preceding Embodiments, wherein the uncorrected distance visual acuity $V_{sc}$ is determined by performing a measurement when the person is

- not using an optical lens to correct the at least one refractive value of the eyer of the person;
- using the optical lens for the eye of the person.

Embodiment 7. The method according to the preceding Embodiment, wherein the at least one optical lens is, particularly, selected from at least one of:

- a spectacle lens;
- a contact lens; or
- an intraocular lens.

Embodiment 8. The method according to any one of the preceding Embodiments, wherein the input data are configured to further comprise a corrected distance visual acuity $V_{cc}$ of the eye of the person.

Embodiment 9. The method according to the preceding Embodiment, wherein the corrected distance visual acuity $V_{cc}$ of the eye of the person is determined from using at least one of:

- a further measurement when the person is using an optical lens for the eye of the person;
- an estimated value, wherein the estimated value is, preferably, determined by using at least one of:

  ◦ an age of the person;
  ◦ an ethnicity of the person;
  ◦ a gender of the person;
  ◦ a known value for the corrected distance visual acuity $V_{cc}$ from a known prescription for the eye of the person;
  ◦ a value for the corrected distance visual acuity $V_{cc}$ of $V_{cc} = 1.0$
  ◦ a statistical model.

Embodiment 10. The method according to any one of the preceding Embodiments, wherein at least one of the uncorrected distance visual acuity $V_{sc}$ or the corrected distance visual acuity $V_{cc}$ of the eye of the person is measured under cycloplegia.

Embodiment 11. The method according to any one of the preceding Embodiments, wherein the meridian direction of the astigmatism of the eye of the person is determined by at least one of:

- a known value from a known prescription;
- a further estimated value; or
- a still further measurement when the person is not using the optical lens for the eye of the person and a further response of the person indicating that the eye of the person exhibits an astigmatism.

Embodiment 12. The method according to the preceding Embodiment, wherein the still further measurement comprises using at least one visual stimulus selected from:

- at least one line or a plurality of lines, particularly wherein the plurality of the lines is grouped in a manner according to which at least two lines of the plurality of lines are oriented in different directions;

- a sun wheel or an astigmatism dial;
- a Raubitschek figure;
- a Gabor patch;
- a grid;
- a cross;
- a Snellen E figure; or
- a Landolt C figure.

Embodiment 13. The method according to the preceding Embodiment, wherein the still further measurement comprises using at least one of:

- an auto-refraction apparatus;
- a wavefront aberrometer.

Embodiment 14. The method according to any one of the preceding Embodiments, wherein the at least one visual stimulus simultaneously aligned in the meridian direction of the astigmatism and the direction perpendicular thereto comprises at least one of:

- a plurality of lines, particularly wherein the plurality of the lines is grouped in a manner according to which at least two lines of the plurality of lines are oriented in the meridian direction of the astigmatism and the direction perpendicular thereto;
- a sun wheel or an astigmatism dial;
- a Raubitschek figure;
- at least two Gabor patches;
- a grid;
- a cross; or
- a Snellen E figure.

Embodiment 15. The method according to any one of the preceding Embodiments, wherein the at least one visual stimulus is displayed to the eye of the person by using at least one of:

- a board; or

- a screen

designed to present the at least one visual stimulus to the eye of the person.

Embodiment 16. The method according to the preceding Embodiment, wherein wherein the at least one visual stimulus isprovided on a surface of the board or projected onto the board.

Embodiment 17. The method according to any one of the two preceding Embodiments, wherein the at least one screen is comprised by at least one of:

- a smartphone;
- a smart watch;
- a smart glass;
- an augmented reality glass;
- a virtual reality glass;
- a desktop computer;
- a laptop;
- a tablet; or
- a smart television.

Embodiment 18. The method according to any one of the three preceding Embodiments, wherein the at least one distance between the eye of the person and the at least one visual stimulus displayed to the eye of the person corresponds to a measured distance between the at least one board or screen and the eye of the person.

Embodiment 19. The method according to any one of the preceding Embodiments, wherein the at least one distance between the eye of the person and the at least one visual stimulus displayed to the eye of the person is determined by using at least one distance measuring device, wherein the distance measuring device is, preferably, selected from at least one of:

- an image-capturing device;
- a sensor-based capturing device, particularly selected from at least one of:

  ◦ an infra-red emitter and detector;
  ◦ a LiDAR module; or
  ◦ a Bluetooth module;

- an ultra-wideband device, particularly comprising at least two communication devices; or
- an independent measurement device, particularly wherein the measurement is performed in a manual fashion by the person, specifically wherein the independent measurement device is a ruler.

Embodiment 20. The method according to any one of the preceding Embodiments, wherein the distance between the eye of the person and the at least one visual stimulus displayed to the eye of the person is altered by at least one of:

- moving the eye of the person towards the at least one board or screen;
- moving the at least one board or screen towards the eye of the person; or
- altering the geometry of the field of view of the eye of the person between the eye and the at least one visual stimulus, particularly by using at least one of:

  ◦ at least one optical lens; or
  ◦ at least one mirror;

- altering at least one of an augmented reality or a virtual reality.

Embodiment 21. The method according to any one of the preceding Embodiments, wherein altering the distance between the eye of the person and the at least one visual stimulus is motivated by at least one command provided to the person, wherein the at least one command comprises a request to the person to alter the distance with regard to the at least one visual stimulus.

Embodiment 22. The method according to the preceding Embodiment, wherein the at least one command is provided to the person by at least one of:

- a visual signal, particularly at least one of piece of textual information or a gesture;
- an audio signal, particularly a voice output or a vibration, specifically generated by using a loudspeaker.

Embodiment 23. The method according to the preceding Embodiment, wherein the piece of textual information is selected from:

- a command requesting the person to move nearer to or further away from the at least one visual stimulus, in particular the at least one board or screen displaying the at least one visual stimulus; or
- a command requesting the person to maintain a position with respect to the at least one visual stimulus, in particular the at least one board or screen displaying the at least one visual stimulus.

Embodiment 24. The method according to any one of the preceding Embodiments, wherein the at least one distance is measured at a point in time at which the person indicates that he or she perceives the at least one visual stimulus in a manner that it exhibits the equivalent blur in the meridian direction of the astigmatism and in the direction perpendicular thereto.

Embodiment 25. The method according to the preceding Embodiment, wherein the point in time is determined by using at least one of:

- an indication of the person or a further person supporting the person, preferably selected from at least one of a parent, a nurse, an optician, or an ophthalmologist, wherein the indication is, particularly, provided by generating at least one of:

   ◦ a visual signal, particularly a gesture;
   ◦ an audio signal, particularly a voice input, specifically provided by using a microphone;
   ◦ a tactile signal, particularly by using at least one of:

       ▪ a keypad; or
       ▪ a touch pad;

- a continuous distance measurement;
- a staircase method;
- a forced choice method.

Embodiment 26. The method according to any one of the preceding Embodiments, wherein determining the at least one refractive value of the eye of the person by evaluating the input data comprises

- determining a value of a blur vector by using the uncorrected distance visual acuity $V_{sc}$ of the eye of the person and, preferably, the corrected distance visual acuity $V_{cc}$;
- determining a spherical equivalent by using the at least one distance determined from the response of the person indicating that the at least one visual stimulus has the equivalent blur in the meridian direction of the astigmatism and in the direction perpendicular thereto; and
- determining the spherical power and the cylindrical power by using the value of the blur vector and the spherical equivalent.

Embodiment 27. The method according to any one of the preceding Embodiments, wherein the value of the blur vector is defined by using equation (1):

$$(blur\ vector)^2 = (sph + 0{,}5 * cyl)^2 + (0{,}5 * cyl)^2, \qquad (1)$$

wherein *sph* denotes the spherical power and *cyl* the cylindrical power.

Embodiment 28. The method according to the preceding Embodiment, wherein determining the value of the blur vector comprises using equations (2) and (3):

$$(blur\ vector)^2 = \left(\frac{1}{V_{rel}-1}\right)^2, \qquad (2)$$

wherein

$$V_{rel} = \frac{V_{sc}}{V_{cc}}, \qquad (3)$$

wherein $V_{sc}$ denotes the uncorrected distance visual acuity of the eye of the person, and wherein $V_{cc}$ denotes the corrected distance visual acuity of the eye of the person.

Embodiment 29. The method according to any one of the two preceding Embodiments, wherein determining the spherical equivalent comprises using equation (4):

$$spherical\ equivalent = 1/d_{DEMB}, \qquad (4)$$

wherein $d_{DEMB}$ denotes the distance determined from the response of the person indicating that the at least one visual stimulus has the equivalent blur in the meridian direction of the astigmatism and in the direction perpendicular thereto.

Embodiment 30. The method according to any one of the two preceding Embodiments, wherein determining the spherical power *sph* and the cylindrical power *cyl* comprises using equations (5) and (6):

$$cyl = 2 * \sqrt{(blur\ vector)^2 + (spherical\ equivalent)^2}, \qquad (5)$$

$$sph = spherical\ equivalent - 0.5 * cyl. \qquad (6)$$

Embodiment 31. The method according to any one of the preceding Embodiments, further comprises controlling an accommodation of the eye of the person, wherein the accommodation of the eye of the person is controlled, in particular by using photoretinoscopy, when the person is viewing at the at least one visual stimulus.

Embodiment 32. The method according to any one of the preceding Embodments, wherein a further eye of the person for which the at least one refractive value is not determined is shielded from the at least one visual stimulus displayed to the eye of the person, particularly by at least one of:

- closing the further eye; or
- covering the further eye, specifically by using a cover, preferably a cardboard or a hand of the person or of a further person.

Embodiment 33. A computer program for determining at least one refractive value of an eye of a person comprising instructions which, when the program is executed by a computer, cause the computer to carry out the method according to any one of the preceding Embodiments.

Embodiment 34. A field device for generating input data for determining at least one refractive value of an eye of a person, wherein the field device is configured to carry out a method for determining at least one refractive value of an eye of a person, comprising the following steps:

a) generating input data configured to comprise

- an uncorrected distance visual acuity $V_{sc}$ of the eye of the person;
- a meridian direction of an astigmatism of the eye of the person;
- a distance between the eye of the person and at least one visual stimulus displayed to the eye of the person, wherein the at least one visual stimulus is simultaneously aligned in the meridian direction of the astigmatism and a direction perpendicular thereto;

wherein the input data are transmitted to a remote device by using a connection interface for determining at least one refractive value of the eye of the person by evaluating the distance between the eye of the person and thr at least one visual stimulus as determined from a response of the person indicating that the at least one visual stimulus has been perceived by the person to have an equivalent blur in the meridian direction of the astigmatism and in the direction perpendicular thereto.

Embodiment 35. The field device according to the preceding Embodiment, wherein the field device is at least one of:

- a smartphone;
- a smart watch;
- a smart glass;
- an augmented reality glass;
- a virtual reality glass;
- a desktop computer;
- a laptop;
- a tablet; or
- a smart television.

Embodiment 36. A remote device for generating outcome data for determining at least one refractive value of an eye of a person, wherein the remote device is configured to carry out a method for determining at least one refractive value of an eye of a person, wherein the remote device is receiving input data provided by a field device, preferably according to any one of the two preceding Embodiments, by using a connection interface, wherein the method is comprising the following step:
b) generating outcome data configured to comprise

- at least one refractive value of the eye of the person, wherein the at least one refractive value of the eye of the person is determined by evaluating the input data,

wherein the input data comprises a distance between the eye of the person and at least one visual stimulus which is determined from a response of the person indicating that the at least one visual stimulus has been perceived by the person to have an equivalent blur in the meridian direction of the astigmatism and in the direction perpendicular thereto.

Embodiment 37. The remote device according to the preceding Embodiment, wherein the remote device is at least one of:

- a server; or
- a cloud server,

and wherein the connection interface is selected from at least one of:

- a network interface controller; or
- a transmitter.

Embodiment 38. A data carrier signal carrying the outcome data generated by a method according to any one of the preceding method Embodiments.

Embodiment 39. A determining device for determining at least one refractive value of an eye of a person, wherein the device is configured to carry out a method for determining at least one refractive value of an eye of a person, wherein the method comprises the following steps:

a) generating input data configured to comprise

- an uncorrected distance visual acuity $V_{sc}$ of the eye of the person;
- a meridian direction of an astigmatism of the eye of the person;
- a distance between the eye of the person and at least one visual stimulus displayed to the eye of the person, wherein the at least one visual stimulus is simultaneously aligned in the meridian direction of the astigmatism and a direction perpendicular thereto;

b) generating outcome data configured to comprise

- at least one refractive value of the eye of the person, wherein the at least one refractive value of the eye of the person is determined by evaluating the input data,

wherein the distance between the eye of the person and the at least one visual stimulus is determined from a response of the person indicating that the at least one visual stimulus has been perceived by the person to have an equivalent blur in the meridian direction of the astigmatism and in the direction perpendicular thereto.

Embodiment 40. The determining device according to the preceding Embodiment, wherein the determining device is at least one of:

- a smartphone;
- a smart watch;
- an augmented reality glass;
- a virtual reality glass;
- a desktop computer;
- a laptop;
- a tablet; or
- a smart television.

Embodiment 41. A method for generating a geometrical model of at least one spectacle lens for producing the at least one spectacle lens, wherein generating the geometrical model of the at least one spectacle lens comprises

- determining the data related to the at least one refractive value by carrying out a method for determining at least one refractive value of an eye of a person in a vision testing procedure, wherein the vision testing procedure comprises at least one test cycle, preferably at least two subsequent test cycles, wherein each test cycle comprises at least the following steps:

a) generating input data configured to comprise

- an uncorrected distance visual acuity $V_{sc}$ of the eye of the person;
- a meridian direction of an astigmatism of the eye of the person;
- a distance between the eye of the person and at least one visual stimulus displayed to the eye of the person, wherein the at least one visual stimulus is simultaneously aligned in the meridian direction of the astigmatism and a direction perpendicular thereto;

b) generating outcome data configured to comprise

- at least one refractive value of the eye of the person, wherein the at least one refractive value of the eye of the person is determined by evaluating the input data,

wherein the distance between the eye of the person and theat least one visual stimulus is determined from a response of the person indicating that the at least one visual stimulus has been perceived by the person to have an equivalent blur in the meridian direction of the astigmatism and in the direction perpendicular thereto; and

- generating the geometrical model of the at least one spectacle lens for the eye of the person by using the outcome data.

Embodiment 42. A method for producing at least one spectacle lens, wherein the at least one spectacle lens is produced by processing at least one lens blank by using the geometrical model of the at least one spectacle lens as generated by using the method according to the preceding Embodiment.

Short description of the Figures

Further optional features and embodiments of the present invention are disclosed in more detail in the subsequent description. Therein, the respective optional features may be implemented in an isolated fashion as well as in any arbitrary feasible combination, as the skilled person will realize. It is emphasized here that the scope of the invention is not restricted by the preferred embodiments. In the Figures:

Figure 1     illustrates an exemplary determining device for determining at least one refractive value of an eye of a person; and

Figure 2     illustrates a method for determining at least one refractive value of an eye of a person.

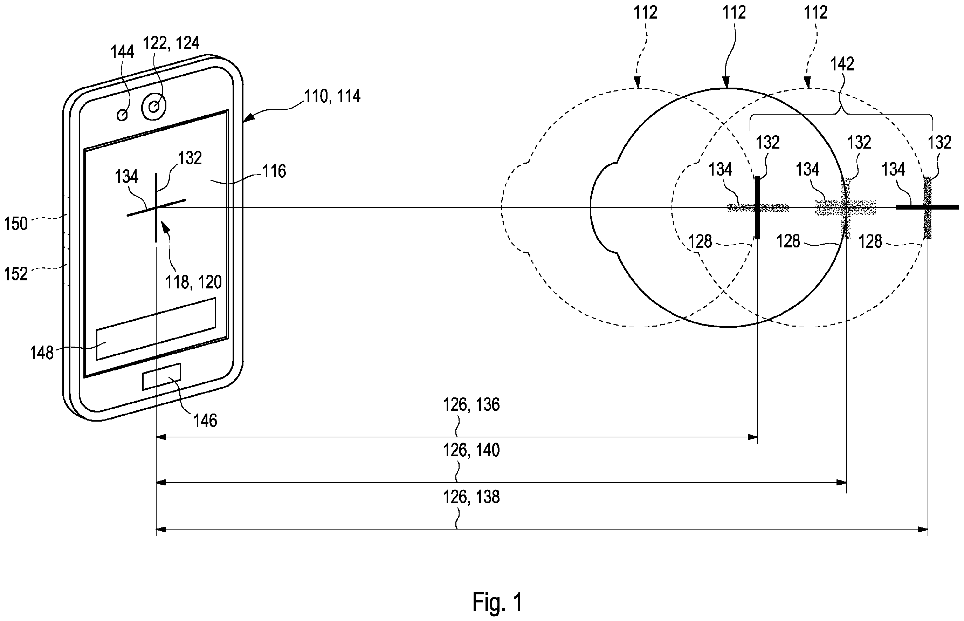

Figure 1 shows an exemplary determining device 110 for determining at least one refractive value 212 of an eye 112 of a person. The determining device 110 is configured to carry out a method 210 for determining the at least one refractive value 212 of the eye 112 of the person in a manner as described in detail in the below. The exemplary determining device 110 as illustrated in Figure 1 is a smartphone 114, which comprises both a field device and a remote device as defined above. Alternatively or in addition, the determining device 110 may be at least one of a smart watch, an augmented reality glass, a virtual reality glass, a desktop computer, a laptop, a tablet, or a smart television; however, using a different kind of remote device may also be feasible. Alternatively or in addition, using separate devices for the field device and the remote device as descriebd above in more detail may alsoe be feasible.

Accordingly, a portion of a method 210 for determining the at least one refractive value 212 of the eye 112 of the person is exemparily presented in Figure 1 as a computer-implemented method. However, the at least one refractive value 212 of the eye 112 of the person may also be determined in a manual fashion (not depicted here) as described above in more detail. In general, the person can be capable of performing all steps of the method 210 for determining the at least one refractive value 212 of the eye 112, as assumed in this exemplary illustration. However, the person may, if desired or required, be accompanied by a further person supporting the person, wherein the accompanying person may, preferably, be selected from a parent and/or a nurse and/or an optician and/or an ophthalmologist.

The exemplary determining device 110 as schematically depicted in Figure 1 comprises a screen 116, which is configured for displaying at least one visual stimulus 118 to the eye 112 of the person. As illustrated there, the exemplary single visual stimulus 118 which is displayed to the eye 112 of the person is a cross 120. However, using at least one different type of visual stimulus 118 may also be feasible, which may, particularly, be selected from more lines, specifically wherein the lines are grouped in a manner according to which at least two lines are oriented in a meridian direction of an astigmatism and a direction perpendicular thereto and/or a sun wheel or an astigmatism dial and/or a Raubitschek figure and/or at least two Gabor patches and/or a grid and/or a Snellen E figure, in particular as described above in more detail.

In addition, the exemplary determining device 110 further comprises a distance measuring device 122. As depicted there, the distance measuring device 122 may be implemented by an image-capturing device, specifically a front camera 124 of the smartphone 114. However using a different type of distance measuring device 122, such as a sensor-based capturing device, in particular an infra-red emitter and detector, a LiDAR module, a Bluetooth module, may also be feasible. As a further alternative or in addition, the distance measuring device 122 may be an ultra-wideband unit, particularly comprising at least two communication devices, or an independent measurement device, particularly when the measurement may be performed in a manual fashion by the person, specifically by using a ruler, especially a pocket rule, an expanding folding rule, or a measuring rod, or a pocket laser rangefinder. By using the distance measuring device 122, at least one distance 126 between the eye 112 of the person, especially between a portion of the retina 128 which comprises the macula, and a location of the at least one visual stimulus 118 on the screen 116 is measured.

As schematically illustrated in Figure 1, the eye 112 of the person has a form, which effects that incident light may not be focusing appropriately on the retina 128 of the eye 112, thus resulting in a defocus of the eye 112. This observation corresponds to the definition of the term "refractive value". In the exemplary arrangement of Figure 1, the at least one refractive value 212 of the eye 112 may comprises a value of a spherical power, a cylindrical power, and a cylinder axis, and, thus, shows an astigmatism. As a result thereof, an image of the cross 120 as the visual stimulus 118 cannot be displayed on the retina 128 of the eye 112 in a clear manner as a whole. Rather, a visual perception by the person of at least a portion of the cross 120 as used herein as the exemplary visual stimulus 118 may, in general, having a blur.

As further schematically illustrated in Figure 1 and a section 130 thereof, the cross 120 as displayed as the visual stimulus 118 on the screen 116 of the smartphone 114 has a vertical line 132 and a horizontal line 134, wherein the vertical line 132 is oriented in a meridian direction of the astigmatism of the eye 112 of the person, while the horizontal line 134 is oriented in a direction perpendicular thereto, or vice versa. As a consequence of this particular orientation of the lines 132, 134 as comprised by the cross 120, a first distance 136 between the screen 116 of the smartphone 114 and the retina 128 of the eye 112 exists, in which only the visual perception of the vertical line 132 by the person is clear, while the horizontal line 134 is having a blur. Similarly, a second distance 138 between the screen 116 of the smartphone 114 and the retina 128 of the eye 112 exists, in which only the visual perception of the horizontal line 134 is clear, while the vertical line 132 is having a blur.

As disclosed in EP 3 730 038 A1, both distances 136, 138 can be used for determining the referactive error of the eye 112 of the person. However, the present invention uses a different approach for determining the referactive error of the eye 112 of the person in a more accurate and reliable fahion. Accordingly, a response of the person that indicates that the cross 120 as the exemplary visual stimulus 118 is perceived by the person in a manner that the vertical line 132 and the horizontal line 134 have an equivalent blur, provides the desired distance 140, which is used here for determining the referactive error of the eye 112 of the person.

In order to determine the desired distance 140, the distance 126 between the retina 128 of the eye 112 of the person and the screen 116 displaying the visual stimulus 118 can be varied within an interval 142 of clear vision, whereby the distance measuring device 122 may, especially in a continuous manner, measure the distance 126, until the person provides the response that the cross 120 is perceived by the person in a manner that both lines 132, 134 have the desired equivalent blur. As an alternative, the desired distance 140 can be measured at a point in time when or immediately after person provides the response that the cross 120 is perceived by the person in the manner of having the desired equivalent blur in both the vertical line 132 and the horizontal line 134.

For a purpose of altering the distance between the eye 112 of the person and the visual stimulus 118 at least one command may provided to the person, thereby requesting the person to alter the distance 126. Herein, the at least one command may be provided to the person by a visual signal, particularly by providing a piece of textual information or a gesture top the person, which may, specifically, be provided by the screen 116 of the smartphone 114. Herein, the piece of textual information may be selected from:

- a command requesting the person to move nearer to or further away from the visual stimulus 118, in particular the screen 116 displaying the visual stimulus 118; or
- a command requesting the person to maintain a position with respect to the visual stimulus 118, in particular to the screen 116 displaying the visual stimulus 118.

However, using a different piece of textual information may also be conceivable. Alternatively or in addition, an audio signal, particularly a voice output or a vibration may be generated, specifically by using a loudspeaker 144 which may, further, be comprised by the smartphone 114.

For a purpose of providing the desired response, the person may generate a visual signal, particularly a gesture, which may, specifically, be received by the front camera 124 of the smartphone 114. Alternatively or in addition, the person may provide an audio signal, particularly a voice input, which may, specifically, be recorder by using a microphone 146 as further comprised by the smartphone 114. As a further alternative or in addition, the person may provide a tactile signal, particularly by using a keypad or a touch pad 148, which may, further, be displayed on the screen 116 of the smartphone 114.

In addition, the exemplary determining device 110, especially the smartphone 118, may further comprise a processing device 150, which is configured to generate input data and outcome data. For this purpose, the determining device 110, especially the smartphone 118, may, still further, comprise a storage device 152, which is configured to store the input data and the outcome data. Alternatively or in additon, the storage device 152 may be or comprise an external storage device (not depicted here) configured for storing the input data and the outcome data or a portion thereof to which the processing device 150 may have access to, preferably via a network, especially via the internet. Accordingly, a value of the desired distance 140 between the eye 112 of the person and the at least one visual stimulus 118 can be stored as one piece of input data in the storage device 152 after it has been determined in the manner as exemplarily decribed above.

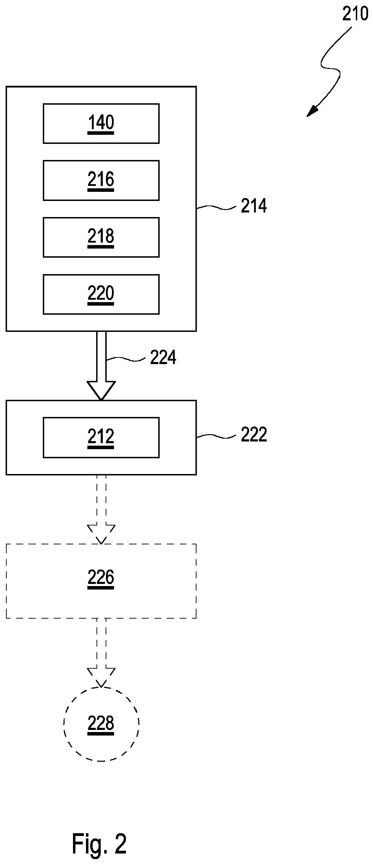

As schematically illustrated in Figure 2, a method 210 for determining the at least one refractive value 212 of the eye 112 of the person comprises generating input data 214, which are configured to comprise a value for each of:

- the desired distance 140 between the eye 112 of the person and the at least one visual stimulus 118, determined in the manner as described above in detail;
- an uncorrected distance visual acuity $V_{sc}$ 216 of the eye 112 of the person;
- optionally, a corrected distance visual acuity $V_{cc}$ 218 of the eye 112 of the person; and
- a meridian direction 220 of the astigmatism of the eye 112 of the person.

As described above in more detail, the uncorrected distance visual acuity $V_{sc}$ 216 corresponds to a visual acuity of the eye 112 of the person to resolve the at least one fine detail in a far point distance of the eye 112 of the person. For determining the uncorrected distance visual acuity $V_{sc}$ 216 of the eye 112 of the person, a respective measurement as known from the prior art may be performed. Alternatively or in addition, a known value for the uncorrected distance visual acuity $V_{sc}$ 216 of the eye 112 of the person may be used for this purpose. Irrespective of the manner used for determining, a value of the uncorrected distance visual acuity $V_{sc}$ 216 of the eye 112 of the person can be stored as a further piece of input data in the storage device 152.

As further described above in more detail, the corrected distance visual acuity $V_{cc}$ 218 of the eye 112 of the person can simply be determind by using an estimated value of $V_{cc} = 1.0$. Such a procedure corresponds to the formula of Raasch et al, see above. As an alternative, the corrected distance visual acuity $V_{sc}$ of the eye 112 of the person can further be determined by using a known value for the corrected distance visual acuity $V_{cc}$ 218 from a known prescription for the eye 112 of the person, preferably together with a further estimated value, in particular an age of the person and/or an ethnicity of the person and/or a gender of the person to be used for further correction, wherein a statistical model may be applied. As a still further alternative or in addition, a further measurement of the eye 112 of the person may be performed, when the person is using an optical lens having at least one known refractive value. For this purpose, again, a respective measurement as known from the prior art may be performed. Irrespective of the manner used for determining, a value of the corrected distance visual acuity $V_{cc}$ 218 of the eye 112 of the person can be stored as a still further piece of input data in the storage device 152.

As still further described above in more detail, the meridian direction 220 of the astigmatism of the eye 112 of the person can be determined by a known value from a known prescription for the eye 112 of the person and/or a further estimated value and/or a still further measurement, when the person is not using the optical lens for the eye 112 of the person and when a further response of the person indicates that the eye 112 of the person exhibits an astigmatism. For performing the still further measurement, at least one further visual stimulus may be used, particularly selected from at least one line or a plurality of lines, particularly wherein the plurality of the lines is grouped in a manner according to which at least two lines of the plurality of lines are oriented in different directions, and/or a sun wheel or an astigmatism dial and/or a Raubitschek figure and/or a Gabor patch and/or a grid and/or a cross and/or a Snellen E figure and/or a Landolt C figure, in particular as described above in more detail. Irrespective of the manner used for determining, the value of the meridian direction 220 of the astigmatism of the eye 112 of the person can be stored as a still further piece of input data in the storage device 152.

In general, the input data 214 may be generated in an arbitrary manner and temporal sequence. Preferably, the values of the uncorrected distance visual acuity $V_{sc}$ 216; optionally of the corrected distance visual acuity $V_{cc}$ 218, and of the meridian direction 220 of the astigmatism may be determined for the eye 112 of the person and stored in the storage device 152 prior to determining the value of the desired distance 140 between the eye 112 of the person and the at least one visual stimulus 118 for the same eye 112 of the person. However, using a different approach may also be feasible, such as determining the value of the desired distance 140 and determining one or more values of the uncorrected distance visual acuity $V_{sc}$ 216, optionally of the corrected distance visual acuity $V_{cc}$ 218, and of the meridian direction 220 of the astigmatism thereafter.

As further schematically depicted in Figure 2, the method 210 comprises generating outcome data 222. As described above in more detail, the outcome data 222 are configured to comprise the at least one refractive value 212 of the eye 112 of the person. For a purpose of determining the at least one refractive value 212 of the eye 112 of the person, the input data 214 are evaluated, in particular by using equations (1) to (6) as presented above. However, using a different approach may also be feasible. For evaluating the input data 214, the values of the desired distance 140, the uncorrected distance visual acuity $V_{sc}$ 216, optionally of the corrected distance visual acuity $V_{cc}$ 218, and of the meridian direction 220 of the astigmatism, which may, irrespective of the manner used for determining, preferably be stored in the storage device 152, may be used as pieces of input data 214 for an algorithm 224 configured for determining the at least one refractive value 212 of the eye 112 of the person as the outcome data. Herein, the algorithm 224 may, preferably, be executed on the processing device 150; however using an external processing device (not depicted here), which may be configured for executing the algorithm 224 or a portion thereof, may also be feasible. In this particular embodiment, the processing device 150 may have access to the external processing device, preferably via a network, especially via the internet. In addition, further arrangements for distributing the executing of the algorithm 224 for evaluating the input data 214 and generating the outcome data 222 may also be conceivable.

As further schematically shown in Figure 2, the outcome data 222 may, preferably, be used for generating a geometrical model 226 of a spectacle lens 228. As further depicted in Figure 2, the geometrical model 226 may, especially, be used for producing the one spectacle lens 228. However, further uses of the outcome data 222 may also be feasible.

List of Reference Signs

| | |
|---|---|
| 110 | determining device |
| 112 | eye |
| 114 | smartphone |
| 116 | screen |
| 118 | visual stimulus |
| 120 | cross |
| 122 | distance measuring device |
| 124 | front camera |
| 126 | distance |
| 128 | retina |
| 130 | section |
| 132 | vertical line |
| 134 | horizontal line |
| 136 | first distance |
| 138 | second distance |
| 140 | (desired) distance |
| 142 | interval of clear vision |
| 144 | loudspeaker |
| 146 | microphone |
| 148 | touch pad |
| 150 | processing device |
| 152 | storage device |
| 210 | method for determining a refractive value of an eye of a person |
| 212 | refractive value |
| 214 | input data |
| 216 | uncorrected distance visual acuity $V_{sc}$ of the eye of the person |
| 218 | corrected distance visual acuity $V_{cc}$ of the eye of the person |
| 220 | meridian direction |
| 222 | outcome data |
| 224 | algorithm |
| 226 | geometrical model |
| 228 | spectacle lens |

**Claims**

1. A method (210) for determining at least one refractive value (212) of an eye (112) of a person, the method (210) comprising the following steps:

   a) generating input data (214) configured to comprise

   - an uncorrected distance visual acuity $V_{sc}$ (216) of the eye (112) of the person;
   - a meridian direction (220) of an astigmatism of the eye (112) of the person;
   - a distance (140) between the eye (112) of the person and at least one visual stimulus (118) displayed to the eye (112) of the person, wherein the at least one visual stimulus (118) is simultaneously aligned in the meridian direction (220) of the astigmatism and a direction perpendicular thereto;

   b) generating outcome data (222) configured to comprise

   - at least one refractive value (212) of the eye (112) of the person, wherein the at least one refractive value (212) of the eye (112) of the person is determined by evaluating the input data (214),

   **characterized in**
   **that** the distance (140) between the eye (112) of the person and the at least one visual stimulus (118) is determined from response of the person indicating that the at least one visual stimulus (118) has been perceived by the person to have an equivalent blur in the meridian direction (220) of the astigmatism and in the direction perpendicular thereto.

2. The method (210) according to the preceding claim, wherein the at least one refractive value (212) of the eye (112) of the person comprises a value of

   - a spherical power;
   - a cylindrical power; and
   - a cylinder axis, wherein the cylinder axis corresponds to the meridian direction (220) of the astigmatism of the eye of the person; and
   - optionally, an addition power, particularly related to a viewing zone for near vision or intermediate vision.

3. The method (210) according to any one of the preceding claims, wherein the uncorrected distance visual acuity $V_{sc}$ (216) is determined by performing a measurement when the person is not using an optical lens to correct the at least one refractive value (212) of the eye (112) of the person.

4. The method (210) according to any one of the preceding claims, wherein the input data (214) are configured to further comprise a corrected distance visual acuity $V_{cc}$ (218) of the eye (112) of the person, wherein the corrected distance visual acuity $V_{cc}$ (218) of the eye (112) of the person is determined by using at least one of:

   - a further measurement when the person is using an optical lens for the eye (112) of the person;
   - an estimated value, wherein the estimated value is, preferably, determined by using at least one of:

     ○ an age of the person;
     ○ an ethnicity of the person;
     ○ a gender of the person;
     ○ a known value for the corrected distance visual acuity $V_{cc}$ (218) from a known prescription for the eye (112) of the person;
     ○ a value for the corrected distance visual acuity $V_{cc}$ (218) of $V_{cc} = 1.0$
     ○ a statistical model.

5. The method (210) according to any one of the preceding claims, wherein the meridian direction (210) of the astigmatism of the eye (112) of the person is determined by at least one of:

   - a known value from a known prescription; or
   - a further estimated value;
   - a still further measurement when the person is not using an optical lens for the eye (112) of the person and a further response of the person indicating that the eye (112) of the person exhibits an astigmatism.

6. The method (210) according to any one of the preceding claims, wherein the at least one distance (140) is measured at a point in time at which the person indicates that he or she perceives the at least one visual stimulus (118) in a manner that it exhibits the equivalent blur in the meridian direction (220) of the astigmatism and in the direction perpendicular thereto, wherein the point in time is determined by using at least one of:

- an indication of the person or a further person supporting the person, preferably selected from at least one of a parent, a nurse, an optician, or an ophthalmologist, wherein the indication is particularly provided by at least one of:

○ a visual signal, particularly a gesture;
○ an audio signal, particularly a voice input, specifically provided by using a microphone (146);
○ a tactile signal, particularly by using at least one of:

■ a keypad (148); or
■ a touch pad;

- a continuous distance measurement;
- a staircase method;
- a forced choice method.

7. The method (210) according to any one of the preceding claims, wherein determining the at least one refractive value (212) of the eye (112) of the person by evaluating the input data comprises

- determining a value of a blur vector by using the uncorrected distance visual acuity $V_{sc}$ (216) of the eye of the person and, preferably, the corrected distance visual acuity $V_{cc}$ (218);
- determining a spherical equivalent by using the at least one distance (140) determined from the response of the person indicating that the at least one visual stimulus (118) has the equivalent blur in the meridian direction (220) of the astigmatism and in the direction perpendicular thereto; and
- determining the spherical power and the cylindrical power by using the value of the blur vector and the spherical equivalent.

8. The method (210) according to the preceding claim, wherein the value of the blur vector is defined by using equation (1):

$$(blur\ vector)^2 = (sph + 0{,}5 * cyl)^2 + (0{,}5 * cyl)^2, \qquad (1)$$

wherein *sph* denotes the spherical power and *cyl* the cylindrical power,
wherein determining the value of the blur vector comprises using equations (2) and (3):

$$(blur\ vector)^2 = \left(\frac{1}{V_{rel}-1}\right)^2, \qquad (2)$$

wherein

$$V_{rel} = \frac{V_{sc}}{V_{cc}}, \qquad (3)$$

wherein $V_{sc}$ denotes the uncorrected distance visual acuity (216) of the eye of the person, and wherein $V_{cc}$ denotes the corrected distance visual acuity (218) of the eye of the person, wherein determining the spherical equivalent comprises using equation (4):

$$spherical\ equivalent = 1/d_{DEMB}, \qquad (4)$$

wherein $d_{DEMB}$ denotes the distance (140) determined from the response of the person indicating that the at least one visual stimulus (118) has the equivalent blur in the meridian direction (220) of the astigmatism and in the direction perpendicular thereto, and wherein determining the spherical power *sph* and the cylindrical power *cyl* comprises using equations (5) and (6):

$$cyl = 2 * \sqrt{(blur\ vector)^2 + (spherical\ equivalent)^2}, \qquad (5)$$

$$sph = spherical\ equivalent - 0.5 * cyl. \qquad (6)$$

9.  A computer program for determining at least one refractive value (212) of an eye (112) of a person comprising instructions which, when the program is executed by a computer, cause the computer to carry out the method (210) according to any one of the preceding claims.

10. A field device for generating input data (214) for determining at least one refractive value (212) of an eye (112) of a person, wherein the field device is configured to carry out at least the following step of a method (210) for determining at least one refractive value (212) of an eye (112) of a person, comprising the following step:

    a) generating input data (214) configured to comprise

    - an uncorrected distance visual acuity $V_{sc}$ (216) of the eye (112) of the person;
    - a meridian direction (210) of an astigmatism of the eye (112) of the person;
    - a distance (140) between the eye (112) of the person and at least one visual stimulus (118) displayed to the eye (112) of the person, wherein the at least one visual stimulus (118) is simultaneously aligned in the meridian direction (220) of the astigmatism and a direction perpendicular thereto;

    **characterized in**
    the input data (214) are transmitted to a remote device by using a connection interface for determining at least one refractive value (212) of the eye (112) of the person by evaluating the distance (140) between the eye (112) of the person and the at least one visual stimulus (118) as determined from a response of the person indicating that the at least one visual stimulus (118) has been perceived by the person to have an equivalent blur in the meridian direction (220) of the astigmatism and in the direction perpendicular thereto.

11. A remote device for generating outcome data (222) for determining at least one refractive value (212) of an eye (112) of a person, wherein the remote device is configured to carry out at least the following step of a method (212) for determining at least one refractive value (212) of an eye (112) of a person, wherein the remote device is receiving input data (214) provided by a field device by using a connection interface, comprising the following step:
    b) generating outcome data (222) configured to comprise

    - at least one refractive value (212) of the eye (112) of the person, wherein the at least one refractive value (212) of the eye (222) of the person is determined by evaluating the input data (214),

    **characterized in**
    the input data (214) comprises a distance (140) between the eye (112) of the person and at least one visual stimulus (118) which is determined from a response of the person indicating that the at least one visual stimulus (118) has been perceived by the person to have an equivalent blur in the meridian direction (220) of the astigmatism and in the direction perpendicular thereto.

12. A data carrier signal carrying the outcome data generated by a method (210) according to any one of the preceding method claims.

13. A determining device (110) for determining at least one refractive value (212) of an eye (112) of a person, wherein the determining device (110) is configured to carry out a method (210) for determining at least one refractive value (212) of an eye (112) of a person, wherein the method (210) comprises the following steps:

    a) generating input data (214) configured to comprise

    - an uncorrected distance visual acuity $V_{sc}$ (216) of the eye of the person;
    - a meridian direction (220) of an astigmatism of the eye of the person;

- a distance (140) between the eye (112) of the person and at least one visual stimulus (118) displayed to the eye (112) of the person, wherein the at least one visual stimulus (118) is simultaneously aligned in the meridian direction (220) of the astigmatism and a direction perpendicular thereto;

b) generating outcome data (222) configured to comprise

- at least one refractive value (212) of the eye (112) of the person, wherein the at least one refractive value (212) of the eye (112) of the person is determined by evaluating the input data (214),

**characterized in**
the distance (140) between the eye (112) of the person and the at least one visual stimulus (118) is determined from a response of the person indicating that the at least one visual stimulus (118) has been perceived by the person to have an equivalent blur in the meridian direction (220) of the astigmatism and in the direction perpendicular thereto.

14. A method for generating a geometrical model (226) of at least one spectacle lens (228) for producing the at least one spectacle lens (228), wherein generating the geometrical model (226) of at least one spectacle lens (228) comprises:

- determining the data related to the at least one refractive value (212) by carrying out a method for determining at least one refractive value (212) of an eye (112) of a person in a vision testing procedure, wherein the vision testing procedure comprises at least one test cycle, preferably at least two subsequent test cycles, wherein each test cycle comprises at least the following steps:

a) generating input data (214) configured to comprise

- an uncorrected distance visual acuity $V_{sc}$ (216) of the eye (112) of the person;
- a meridian direction (220) of an astigmatism of the eye (112) of the person;
- a distance (140) between the eye (112) of the person and at least one visual stimulus (118) displayed to the eye (112) of the person, wherein the at least one visual stimulus (118) is simultaneously aligned in the meridian direction (220) of the astigmatism and a direction perpendicular thereto;

b) generating outcome data (222) configured to comprise

- at least one refractive value (212) of the eye (112) of the person, wherein the at least one refractive value (212) of the eye (112) of the person is determined by evaluating the input data (214),

wherein the distance (140) between the eye (112) of the person and the at least one visual stimulus (118) is determined from a response of the person indicating that the at least one visual stimulus (118) has been perceived by the person to have an equivalent blur in the meridian direction (220) of the astigmatism and in the direction perpendicular thereto; and

- generating the geometrical model (226) of the at least one spectacle lens (228) for the eye (112) of the person by using the outcome data (222).

15. A method for producing at least one spectacle lens (228), wherein the at least one spectacle lens (228) is produced by processing at least one lens blank by using the geometrical model (226) of the at least one spectacle lens (228) as generated by using the method according to the preceding claim.

Fig. 1

Fig. 2

Europäisches
Patentamt

European
Patent Office

Office européen
des brevets

# EUROPEAN SEARCH REPORT

**Application Number**

EP 23 21 0413

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2019/307324 A1 (LIMON OFER [IL]) 10 October 2019 (2019-10-10) * the whole document * | 1-15 | INV. G02C7/02 |
| A,D | EP 3 730 038 B1 (VISIONAPP SOLUTIONS S L [ES]) 8 November 2023 (2023-11-08) * paragraph [0045] – paragraph [0056] * * claim 1 * * claim 5 * | 1-15 | |
| A | US 2023/218160 A1 (LAI SHUI T [US]) 13 July 2023 (2023-07-13) * paragraph [0114] * | 1-15 | |
| A | KR 102 470 341 B1 (SPEQS LTD [AU]) 24 November 2022 (2022-11-24) * paragraph [0202] – paragraph [0210] * * claim 3 * | 1-15 | |
| A | US 2023/053457 A1 (BOUTINON STEPHANE [FR] ET AL) 23 February 2023 (2023-02-23) * paragraph [0116] * | 1-15 | TECHNICAL FIELDS SEARCHED (IPC) |
| A | JP 2016 502890 A (RODENSTOCK GMBH) 1 February 2016 (2016-02-01) * paragraph [0055] – paragraph [0062] * | 1-15 | G02C A61B |

The present search report has been drawn up for all claims

| Place of search | Date of completion of the search | Examiner |
|---|---|---|
| Munich | 10 April 2024 | Alvazzi Delfrate, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 0413

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2024

| Patent document cited in search report | | | Publication date | Patent family member(s) | | | Publication date |
|---|---|---|---|---|---|---|---|
| US 2019307324 | A1 | | 10-10-2019 | AU | 2014276340 | A1 | 24-12-2015 |
| | | | | BR | 112015030406 | A2 | 25-07-2017 |
| | | | | CA | 2914456 | A1 | 11-12-2014 |
| | | | | CN | 105764405 | A | 13-07-2016 |
| | | | | CN | 110251066 | A | 20-09-2019 |
| | | | | DK | 3003121 | T3 | 16-08-2021 |
| | | | | EP | 3003121 | A1 | 13-04-2016 |
| | | | | ES | 2886136 | T3 | 16-12-2021 |
| | | | | JP | 6453859 | B2 | 16-01-2019 |
| | | | | JP | 6612959 | B2 | 27-11-2019 |
| | | | | JP | 2016523132 | A | 08-08-2016 |
| | | | | JP | 2019069180 | A | 09-05-2019 |
| | | | | KR | 20160017079 | A | 15-02-2016 |
| | | | | PT | 3003121 | T | 06-09-2021 |
| | | | | RU | 2706372 | C1 | 18-11-2019 |
| | | | | RU | 2015152290 | A | 14-07-2017 |
| | | | | US | 2016120402 | A1 | 05-05-2016 |
| | | | | US | 2017079523 | A1 | 23-03-2017 |
| | | | | US | 2019307324 | A1 | 10-10-2019 |
| | | | | WO | 2014195951 | A1 | 11-12-2014 |
| EP 3730038 | B1 | | 08-11-2023 | AU | 2020260698 | A1 | 21-10-2021 |
| | | | | BR | 112021018834 | A2 | 03-05-2022 |
| | | | | CA | 3131765 | A1 | 29-10-2020 |
| | | | | CN | 113993441 | A | 28-01-2022 |
| | | | | EP | 3730038 | A1 | 28-10-2020 |
| | | | | EP | 4275587 | A2 | 15-11-2023 |
| | | | | JP | 2022529893 | A | 27-06-2022 |
| | | | | US | 2022151488 | A1 | 19-05-2022 |
| | | | | WO | 2020216732 | A1 | 29-10-2020 |
| US 2023218160 | A1 | | 13-07-2023 | US | 2023218160 | A1 | 13-07-2023 |
| | | | | WO | 2020172203 | A1 | 27-08-2020 |
| | | | | WO | 2021167647 | A1 | 26-08-2021 |
| KR 102470341 | B1 | | 24-11-2022 | AU | 2020286342 | A1 | 03-06-2021 |
| | | | | CN | 112043230 | A | 08-12-2020 |
| | | | | JP | 7166473 | B2 | 07-11-2022 |
| | | | | JP | 2022526867 | A | 26-05-2022 |
| | | | | KR | 20220024411 | A | 03-03-2022 |
| | | | | US | 2022304572 | A1 | 29-09-2022 |
| | | | | WO | 2020243771 | A1 | 10-12-2020 |
| US 2023053457 | A1 | | 23-02-2023 | CN | 114828730 | A | 29-07-2022 |
| | | | | EP | 4076139 | A1 | 26-10-2022 |
| | | | | JP | 2023506515 | A | 16-02-2023 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**          EP 23 21 0413

10-04-2024

| Patent document cited in search report | Publication date | Patent family member(s) | | Publication date |
|---|---|---|---|---|
| | | KR | 20220116159 A | 22-08-2022 |
| | | US | 2023053457 A1 | 23-02-2023 |
| | | WO | 2021122640 A1 | 24-06-2021 |
| JP 2016502890 A | 01-02-2016 | DE | 102013000295 A1 | 10-07-2014 |
| | | EP | 2943114 A1 | 18-11-2015 |
| | | JP | 6781802 B2 | 04-11-2020 |
| | | JP | 6842499 B2 | 17-03-2021 |
| | | JP | 2016502890 A | 01-02-2016 |
| | | JP | 2019166345 A | 03-10-2019 |
| | | JP | 2019166346 A | 03-10-2019 |
| | | US | 2015313463 A1 | 05-11-2015 |
| | | WO | 2014108167 A1 | 17-07-2014 |

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20190307324 A1
- US 20200397279 A1
- EP 3730038 A1
- US 20190301324 A1

### Non-patent literature cited in the description

- **THOMAS W. RAASCH**. Spherocylindrical Refractive Errors and Visual Acuity. *Optometry and Vision Science*, vol. 72 (4), 272-275
- **RALF BLENDOWSKE**. *Unaided Visual Acuity and Blur: A Simple Model*
- Ophthalmic optics - Visual acuity testing - Standard and clinical optotypes and their presentation. *ISO 8596:2017*